(19) Europäisches Patentamt
European Patent Office
Office européen des brevets

(11) Publication number: **0 294 183 B1**

# (12) EUROPEAN PATENT SPECIFICATION

(45) Date of publication of patent specification: **30.03.94**

(51) Int. Cl.⁵: **C07D 211/32**, C07D 211/22, C07D 211/18, C07D 285/24, C07D 401/06, A61K 31/445, A61K 31/54

(21) Application number: **88305012.2**

(22) Date of filing: **01.06.88**

The file contains technical information submitted after the application was filed and not included in this specification

## (54) Use of ethylamine derivatives as antihypertensive agents.

(30) Priority: **02.06.87 JP 138405/87**

(43) Date of publication of application:
**07.12.88 Bulletin 88/49**

(45) Publication of the grant of the patent:
**30.03.94 Bulletin 94/13**

(84) Designated Contracting States:
**CH DE FR GB IT LI**

(56) References cited:
**EP-A- 292 735**
**EP-A- 2 293 91**
**US-A- 3 829 433**
**US-A- 4 559 349**

(73) Proprietor: **AJINOMOTO CO., INC.**
**5-8, Kyobashi 1-chome, Chuo-ku**
**Tokyo 104(JP)**

(72) Inventor: **Syoji, Masataka**
**No. 1-1 Suzuki-cho**
**Kawasaki-ku**
**Kawasaki-shi Kanagawa-ken(JP)**

Inventor: **Eguchi, Chikahiko**
**No. 1-1 Suzuki-cho**
**Kawasaki-ku**
**Kawasaki-shi Kanagawa-ken(JP)**
Inventor: **Toyota, Kozo**
**No. 1-1 Suzuki-cho**
**Kawasaki-ku**
**Kawasaki-shi Kanagawa-ken(JP)**
Inventor: **Yoshimoto, Ryota**
**No. 1-1 Suzuki-cho**
**Kawasaki-ku**
**Kawasaki-shi Kanagawa-ken(JP)**
Inventor: **Koyama, Yosikatsu**
**No. 1-1 Suzuki-cho**
**Kawasaki-ku**
**Kawasaki-shi Kanagawa-ken(JP)**
Inventor: **Domoto, Hideki**
**No. 1-1 Suzuki-cho**
**Kawasaki-ku**
**Kawasaki-shi Kanagawa-ken(JP)**
Inventor: **Kamimura, Akira**
**No. 1-1 Suzuki-cho**
**Kawasaki-ku**
**Kawasaki-shi Kanagawa-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 294 183 B1

Rank Xerox (UK) Business Services
(3.10/3.09/3.3.3)

(74) Representative: **Armitage, Ian Michael et al**
**MEWBURN ELLIS**
**2 Cursitor Street**
**London EC4A 1BQ (GB)**

## Description

The present invention relates to the use of particular ethylamine derivatives as antihypertensive drugs.

Hypertension is a common complaint, whose frequency increases with age. In addition, hypertension is an important factor in cerebral apoplexy and cardiopathy which are also frequent causes of death. Accordingly, an antihypertensive drug is considered to be one of the most important medicines for geriatric diseases.

In 90% or more of hypertensive patients the cause of the hypertension is not clear, and patients are required to take an antihypertensive drug all their lives, and therefore the antihypertensive drug has to have high safety and reliable pharmaceutical activity against hypertension, as well as being long-acting.

Recently, serotonin-antagonistic antihypertensives have been reported, for example in Journal of Cardiovascular Pharmacology, 7 (Suppl. 7), S110-S111 (1985). Such antihypertensives, however, are not always satisfactory in view of their antihypertensive mode of action and their low activity. Such medicines have also been reported to be effective also against various diseases caused by serotonin, for example, thrombosis, ulcer and the like, but the effect against such diseases is not clearly established. Furthermore many of such antihypertensives and their synthetic intermediates are rather insoluble in organic solvents or are produced by complicated and troublesome procedures.

The present invention provides the use of particular ethylamine derivatives represented by the following formula in the preparation of antihypertensive medicaments. Such compounds exhibit serotonin-antagonistic activity and antihypertensive activity and can be prepared simply and industrially advantageously.

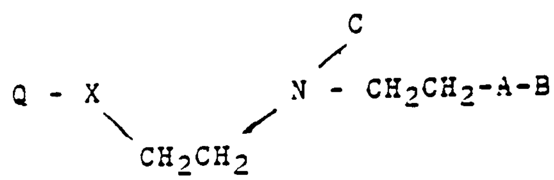
(I)

In the above formula (I):

The structure represented by Q in the formula is any one of the following:

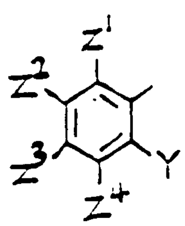
(II)

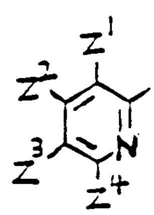
(III)

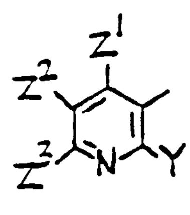
(IV)

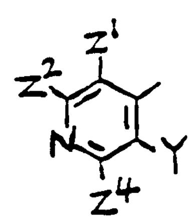
(V)

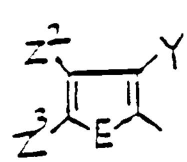
(VI)

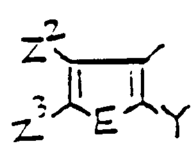
(VII)

wherein E is a nitrogen atom, -NH or substituted -NH, an oxygen atom or a sulfur atom: Y, $Z^1$, $Z^2$, $Z^3$ and $Z^4$ are independently selected from hydrogen and alkyl of from 1 to 6 carbon atoms, alkyl of from 1 to 6 carbon atoms substituted with one or more halogen, oxygen, sulfur, nitrogen or phosphorus atoms, aralkyl of from 6 to 12 carbon atoms aralkyl of from 6 to 12 carbon atoms substituted with one or more halogen, oxygen, sulfur, nitrogen or phosphorus atoms, aryl of from 6 to 12 carbon atoms and aryl of from 6 to 12

carbon atoms substituted with one or more halogen, oxygen, sulfur, nitrogen or phosphorus atoms: Y is optionally bonded to X to provide a linking group in which the linkage is formed by an alkylene bridge or an alkylene bridge substituted with one or more halogen, oxygen, sulfur, nitrogen or phosphorus atoms and/or at least one oxygen, sulfur or nitrogen atom; and $Z^1$ and $Z^2$, $Z^2$ and $Z^3$, or $Z^3$ and $Z^4$ are optionally bonded to each other to provide a linking group in which the linkage is formed by an alkylene bridge or an alkylene bridge substituted with one or more halogen, oxygen, sulfur, nitrogen or phosphorous atoms and/or at least one oxygen, sulfur or nitrogen atom.

Specific examples of the substituent Q are as follows:

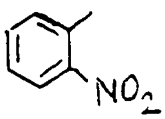
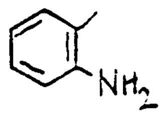
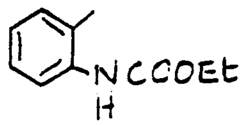
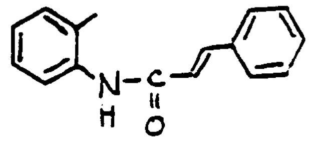
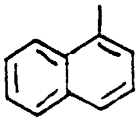
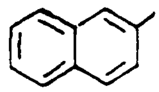
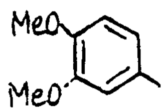
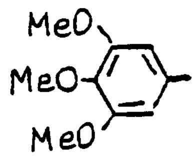
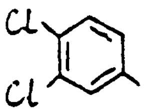
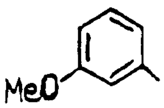
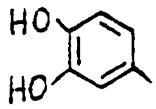
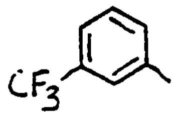
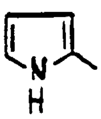
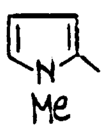
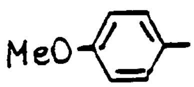
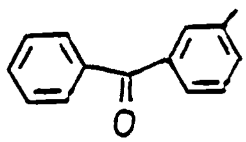
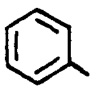
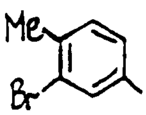
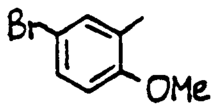
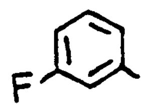
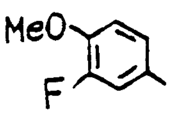
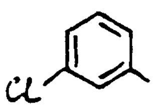
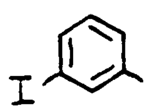
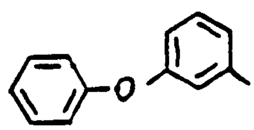
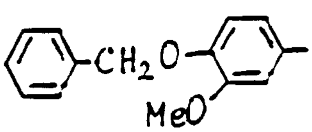
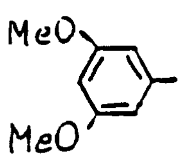
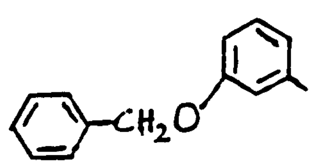

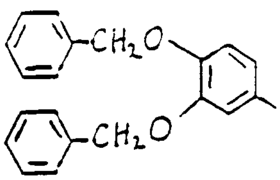
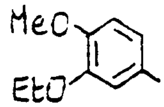
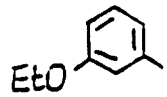

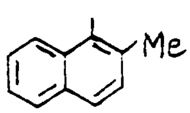
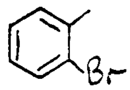
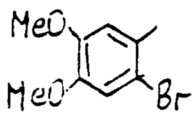
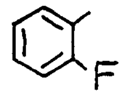

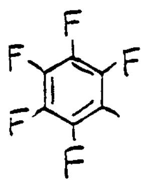
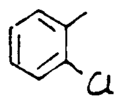
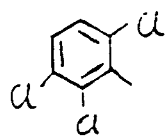
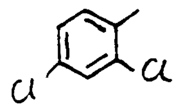
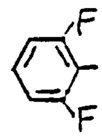

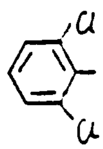
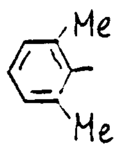
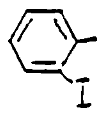
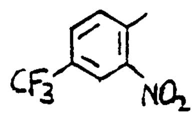
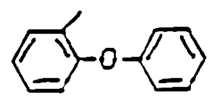

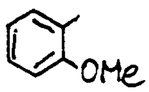
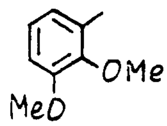
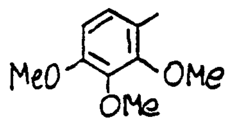
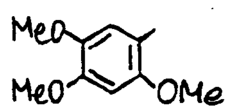

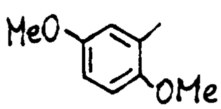
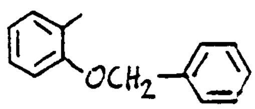
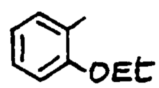
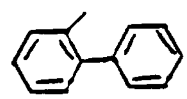

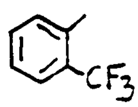
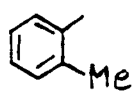
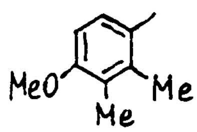
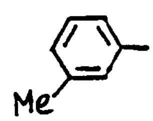
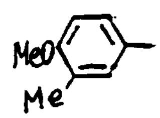

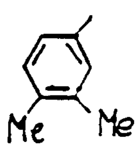
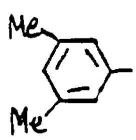
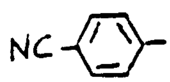
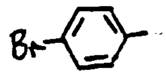

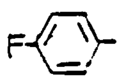
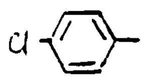
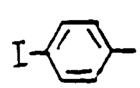
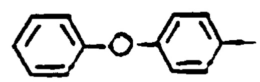

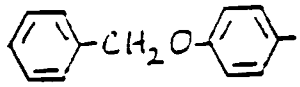 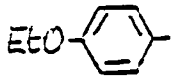 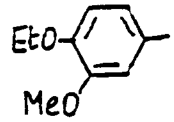

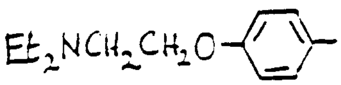 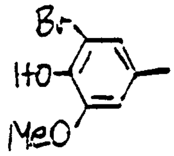 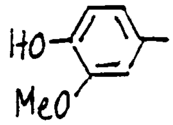

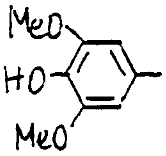 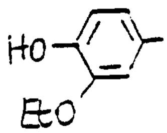 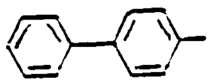 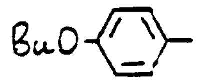

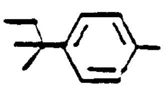 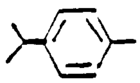 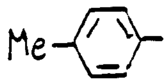 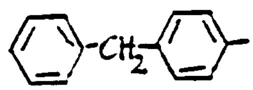

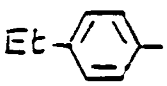 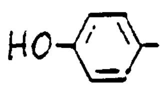 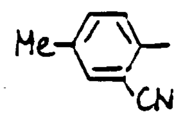 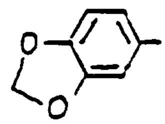

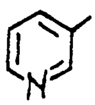 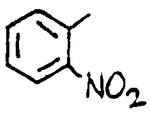 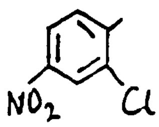 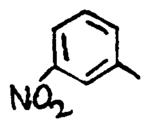

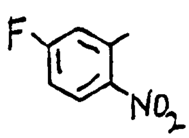 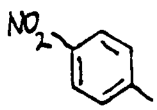 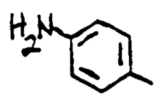 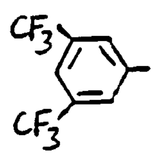

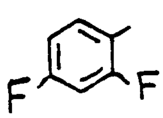 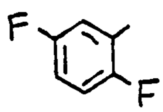 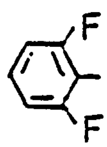 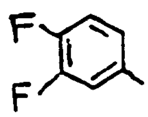

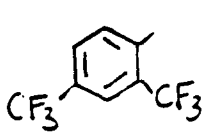 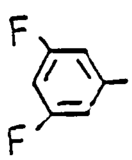 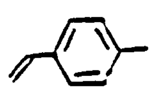 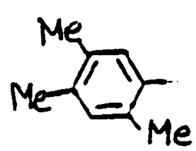

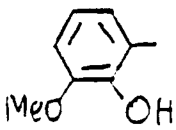 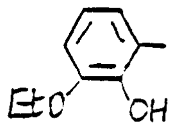 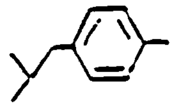

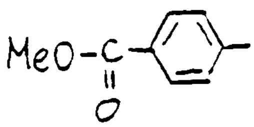 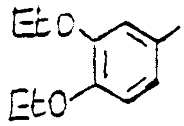 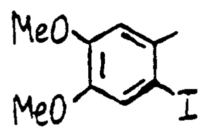

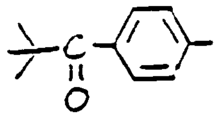 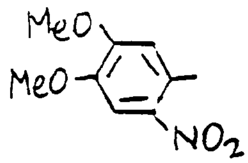 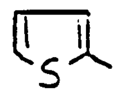

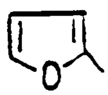 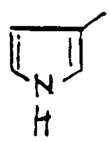 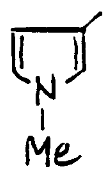 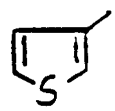

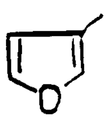 .

C is hydrogen, methyl, ethyl, propyl, butyl, pentyl or hexyl, or is bonded to A to form a methylene, ethylene or propylene bridge.

The structure represented by the combined group -A-B in the formula is selected from the following:

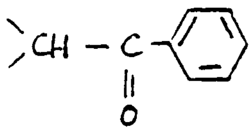 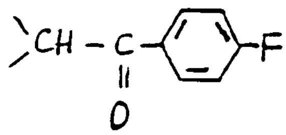 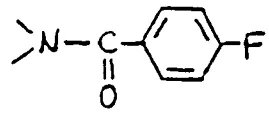

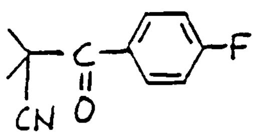 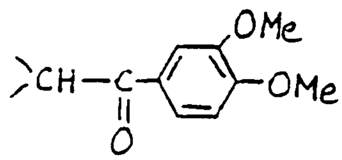

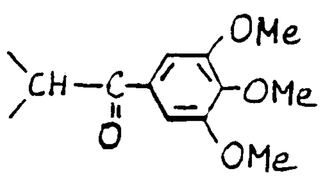 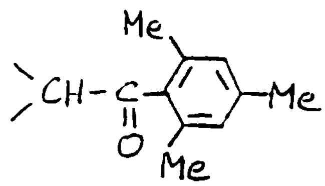

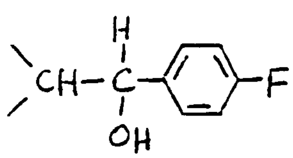 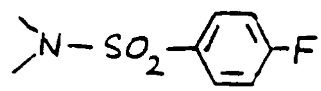

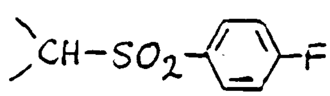 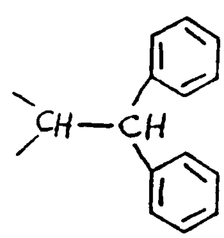

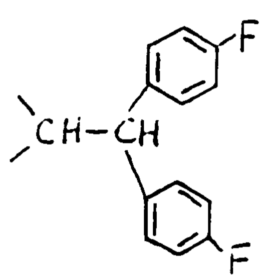 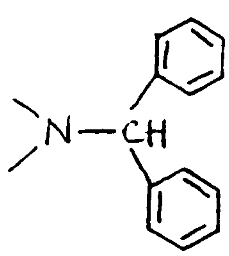

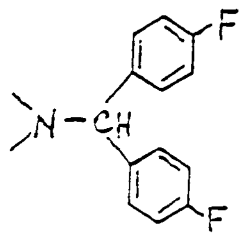

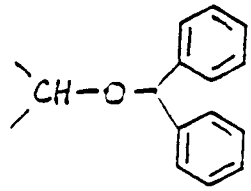

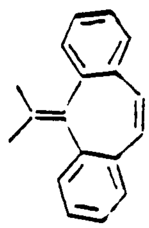

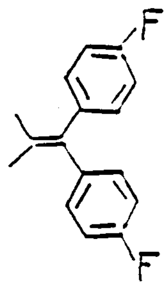

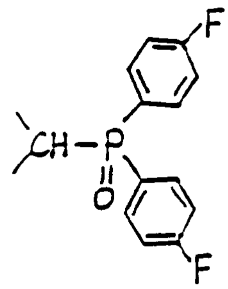

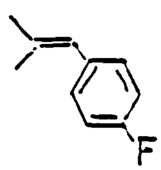

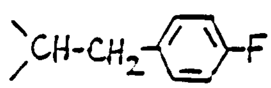

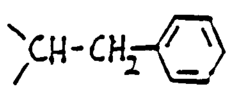

In the above formulae it will be seen that A is represented by any of the following: >CH-, >C=, >N- or >C(CN)-, in which one of the bonds shown is linked to the group C in Formula I, or carries hydrogen. B, on the other hand, can be regarded as optionally substituted aryl or aralkyl, which can be linked to A through a substituent such as sulfonyl or carbonyl.

The linking group X is an organic group selected from the following (VIII-XI):

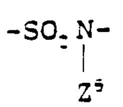

(VIII)

$-S(CH_2)_n-$

(IX)

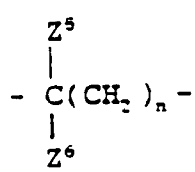

(X)

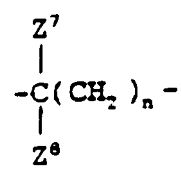

(XI)

wherein, n represents an integer from 0 to 9; $Z^5$ and $Z^6$ are independently hydrogen, alkyl or aralkyl, or alkyl or aralkyl substituted with one or more hetero atoms and/or with one or more substituents optionally containing one or more hetero atoms, $Z^7$ is an alkyl group of from 1 to 20 carbon atoms, or an alkyl group of from 1 to 20 carbon atoms substituted with one or more hetero atoms and/or with one or more substituents optionally containing one or more hetero atoms; and $Z^8$ is hydrogen, cyano, amino, nitro, carboxyl, ester or aminocarbonyl. The ester of $Z^8$ may for example be alkoxycarbonyl or alkoxysulfonyl.

It will be apparent, therefore, that X can consist merely of an optionally substituted alkylene group (formula XI), or form a linkage solely through a nitrogen and/or sulfur atom (formula VIII), or a combination of these (formula IX).

The ethylamine derivative used in accordance with the present invention may be in the form of a salt thereof.

Of course, when the salt of the said derivative is incorporated into an antihypertensive medicament in accordance with the present invention, it should be in the form of a pharmaceutically acceptable salt.

The ethylamine derivative defined above is advantageous as an antihypertensive for treating hypertensive mammals including humans. This can be administered perorally in the form of a tablet, capsule or elixir, or parenterally in the form of a sterile solution or suspension, for the purpose of reducing blood pressure. The ethylamine derivative can be administered to patients or animals which are to be treated generally several times each in a unit dosage of from 0.2 to 500 mg/patient or animal, and accordingly, the total dosage of the derivative may be from 1 to 2000 mg/patient or animal/day. Of course, the amount of the dosage may be varied in accordance with the condition of the disease, the weight of the patient or animal and other factors which are considered appropriate by one skilled in the art.

The ethylamine derivative can also be used together with another antihypertensive agent. As examples of such agents which can be used together with the derivative of the invention, there may be mentioned $\alpha_1$-antagonistssuch as prozosin, a calcium-antagonist such as nifedipine, nicardipine, diltiagen and verapamil, or a convertase inhibitor such as captoril or enalapril.

The above-mentioned typical combinations may be formulated as a pharmaceutical composition in conventional manner. From about 0.2 to about 500 mg of the ethylamine derivative or a physiologically acceptable salt thereof or a mixture thereof is blended together with a physiologically acceptable vehicle, carrier, extender, binder, antiseptic, stabilizer, flavour and the like, in amounts as required for conventional pharmaceutical preparations.

Examples of pharmaceutical additives to be used for preparation of tablets, capsules and the like are: binders such as tragacanth, gum arabic, corn starch or gelatin; vehicles such as fine crystalline cellulose; extenders such as corn starch, pre-gelatinised starch or alginic acid; sweeteners such as sucrose, lactose or saccharin; flavours such as peppermint, an oil from Gaulthenia adenothrix Maxim or cherry. When the preparation is in the form of a capsule, this may further contain a liquid carrier such as a fat and oil, in addition to the above-mentioned materials. Other various materials can further be employed so as to form coated pills or to vary the physical form of the preparation by a different method. For example, tablets can be coated with shellac, sugar or both. A syrup or elixir can contain the active compound together with sucrose as a sweetener, methyl- or propyl-paraben as an antiseptic, a dye, and cherry or orange essence as a flavour.

A sterile composition for injection can be prepared in conventional manner, for example, by dissolving or suspending the active substance in a vehicle such as distilled water for injection, together with a natural vegetable oil such as sesame oil, coconut oil, peanut oil, cotton seed oil, or a synthetic fat vehicle such as ethyl oleate. If desired, a buffer, an antiseptic, an antioxidant or the like can be incorporated into the composition.

The present invention will be further illustrated by reference to the following examples:

Example 1: Synthesis of 1-[2-(2-nitrobenzenesulfonyl)aminoethyl]-4-(4-fluorobenzoyl)piperidine:

(a) Synthesis of (2-nitrobenzenesulfonyl)aminoethyl bromide:

20 ml of a dichloroethane solution of 21.0 g (200 mmol) of triethylamine was added dropwise to 80 ml of a dichloroethane solution containing 22.1 g (100 mmol) of 2-nitrobenzenesulfonyl chloride and 20.4 g (100 mmol) of 2-bromoethylamine hydrobromide, with ice-cooling. After completion of the dropwise addition, the whole was stirred overnight at room temperature. After the reaction, the reaction mixture was washed twice with 100 ml of 1 N hydrochloric acid solution and then once with 100 ml of water, and then the organic layer was dried with an anhydrous sodium sulfate.

The solvent was evaporated under reduced pressure to obtain the above-titled compound (a) in crystalline form.

Yield: 29.5 g (95.4 mmol), 95%.

Thin Layer Chromatography: (TLC)

(AcOEt : n - $C_6H_{14}$ = 1 : 4 )

$R_f$ = 0.70

Mass Spectrum: (MS)

(FAB, m/z)

309 (M + $H^+$)

Nuclear Magnetic Resonance Spectrum: ($^1$H-NMR)

($TMS/CDCl_3$, $\delta$/ppm)

3.45-3.65 (4H,m), 6.75 (1H,z) 7.53-8.08 (4H,m)

(b) Synthesis of 1-[2-(2-nitrobenzenesulfonyl)aminoethyl]-4-(4-fluorobenzoyl)piperidine:

A mixture comprising 9.27 g (30 mmol) of 2-nitrobenzenesulfonyl)aminoethyl bromide, 7.31 g (30 mmol) of 4-fluorobenzoylpiperidine hydrochloride, 8.48 g (80 mmol) of sodium carbonate and 100 ml of methyl isobutyl ketone was stirred overnight in an oil bath.

After the reaction, 50 ml of water was added to the reaction mixture for washing, and then the organic layer was isolated and the solvent was evaporated under reduced pressure.

After purification by silica gel column chromatography (eluent: chloroform/methanol = 5/1), the resulting product was converted into its hydrochloride with 4 N hydrogen chloride/dioxane solution.

Yield: 10.5 g (22.2 mmol), 76 %.

TLC

($CHCl_3$: MeOH = 9:1)

$R_f$ = 0.81

MS (FAB, m/z)

436 ($M + H^+$)

$^1$H-NMR (TMS/DMSO-$d_6$, $\delta$/ppm)

1.80-2.05 (5H,m), 3.04-3.35 (6H,m) 3.56-3.65 (2H,m), 3.65-3.75 (1H,m) 7.90-7.96 (2H,m), 7.40 (2H,dd,J=8, 8Hz), 8.45 (1H,bz), 8.02-8.13 (34H,m)

Example 2: Synthesis of 1-[2-(2-aminobenzenesulfonyl)aminoethyl]-4-(4-fluorobenzoyl)piperidine:

A suspension of 1-[2-(2-nitrobenzenesulfonyl)aminoethyl]-4-(4-fluorobenzoyl)piperidine (see Example 1) in 160 ml of acetic acid was heated at 90 °C and then 8 g of iron (powder) was gradually added thereto over a period of 10 minutes. The reaction solution became black, and after this was stirred for a further 10 minutes, white crystals were precipitated. After cooling, the reaction solution was filtered, and the residue was washed with methanol. The solvent was evaporated from the resulting filtrate, and 4 N hydrogen chloride/dioxane solution was added to the residue. Thus the above-entitled compound was obtained in the form of the hydrochloride.

Yield: 65.8g (13.8 mmol), 71%

TLC

($CHCl_3$; MeOH = 5:1)

$R_f$ = 0.40

MS (FAB, m/z)

406 ($M+H^+$)

$^1$H-NMR (TMS/DMSO - $d_6$, $\delta$/ppm)

1.84-2.01 (5H,m), 3.02-3.20 (6H,m) 3.48-3.55 (2H,m), 3.64-3.72 (1H,m) 4.72 (1H,bz), 6.63 (1H, dd, J=8, 8Hz) 6.84 (1H, dd, J=8 Hz), 7.30 (1H, dd, J=8, 8Hz), 7.39 (2H, dd, J=6, 6Hz) 7.53 (1H, dd, J=8 Hz), 7.98 (1H, bz) 8.08 (2H, dd, J=8, 6 Hz)

Example 3: Synthesis of 1-[2-(2-ethoxycarbonylaminobenzenesulfonyl) aminoethyl]-4-(4-fluorobenzoyl)-piperidine:

30 ml of a chloroform solution containing 3.65 g (9.00 mmol) of 1-[2-(2-aminobenzenesulfonyl)-aminoethyl]-4-(4-fluorobenzyl)piperidine, 0.16 g (0.90 mmol) of 4-piperidinopyridine and 0.91 g (9.00 mmol) of triethylamine was blended and 0.976 g (9.00 mmol) of ethyl chloroformate was added dropwise. After the completion of the reaction, the reaction mixture was stirred for three hours and then extracted twice with 50 ml of 1 N hydrochloric acid and then once with 50 ml of water in that order. Then, the resulting organic layer was dried with anhydrous magnesium sulfate.

The solvent was evaporated and the residue was purified by column chromatography (eluent: ethyl acetate/ethanol = 20/1). The above-titled compound was obtained as an oily product.

Yield: 3.18 g (6.67 mmol), 74.1%.

TLC

(AcOEt: EtOH = 20 :1)

$R_f$ = 0.87

MS (FD, m/z)

478 ($M + H^+$)

$^1$H-NMR (TMS/DMSO - $d_6$, $\delta$/ppm)

1.12 (3H, t, J = 8 Hz), 1.82-2.10 (5H, m), 3.12-3.27 (2H,m), 3.65-3.77 (4H,m), 4.10 (2H, q, J = 8Hz), 4.23-4.30 (2H,m), 6.16 (1H, bz), 6.68 (1H, dd, J = 8, 8 Hz), 6.89 (1H, d, J = 8 Hz), 7.36 (1H, dd, J = 8.8 Hz), 7.40 (2H, dd, J = 8, 6 Hz), 7.59 (1H, d, J = 8 Hz), 8.10 (2H, dd, J = 8, 6 Hz)

Example 4: Synthesis of 5,6-benzo-2,4-diaza-2-[2-[(4-fluorobenzyl)piperidin-1-yl]ethyl]tetrahydrothiopyrane-1,1-dioxide:

20 ml of a pyridine solution of 1.10 g (2.30 mmol) of 4-[2-(2-ethoxycarbonylaminobenzenesulfonyl)-aminoethyl]-1-(4-fluorobenzoyl)piperidine (refer to Example 3) was heated under reflux for 15 hours. After completion of the reaction, the solvent was evaporated, and dichloroethaneether was added to the residue for crystallization. The resulting crystals were filtered, washed with ether and then dried under reduced pressure to obtain the above-titled compound.

Yield: 0.31 g (0.72 mmol), 32 %.

TLC

($CHCl_3$: MeOH = 9:1)

$R_f$ = 0.66

MS (FD, m/z)

432 ($M+H^+$)

$^1$H-NMR(TMS/$CDCl_3$, $\delta$/ppm)

1.78-1.98 (5H,m), 2.38-2,52 (2H,m) 2.82-2.92 (2H,m), 3.05-3.14 (2H,m) 3.25 (1H, bz), 4.20 (2H, t, J = 7 Hz) 7.13 (2H, dd, J = 8, 8 Hz), 7.20 (1H, d, J = 8 Hz), 7.26 (1H, dd, J = 8, 8 Hz) 7.57 (1H, d, J = 8, 8 Hz), 7.80 (1H, d, J = 8 Hz), 7.94 (2H, dd, J = 8, 8 Hz)

Example 5: Synthesis of 2-[3,4-dimethoxyphenyl]-5-[4-(4-fluorobenzoyl)-1-piperidinyl]-2-isopropylvaleronitrile hydrochloride:

(a) Synthesis of 2-(3,4-dimethoxyphenyl)-2-isopropylacetonitrile:

A three-neck flask equipped with an argon balloon and two dropping funnels was flushed with argon and 6.6 g (90.17 mol) of sodium amide was put thereinto. After again flushing with argon, 30 ml of 1,2-dimethoxyethane was added. 25 g (0.14 mol) of 3,4-dimethoxyphenyl-acetonitrile was put in another flask, which was then flushed with argon, and 25 ml of 1, 2-dimethoxyethane was added. The solution was transferred into the dropping funnel with a cylinder. The 3,4-dimethoxyphenylacetonitrile solution was added dropwise to the sodium amide suspension with stirring under ice-cooling. The ice-bath was removed and then the whole was stirred for a further 1 hour. Next, the ice-bath was again applied to the reaction solution and a 1,2-dimethoxyethane solution (17 ml) containing 17.4 ml (0.19 mol) of isopropyl bromide was added dropwise, and then the whole was stirred overnight at room temperature. The reaction mixture was poured into ice-water, extracted with ether and then washed with aqueous 1N sodium hydroxide solution, water, aqueous 5% citric acid solution and water, in that order. The ether layer was dried with anhydrous magnesium sulfate and the solvent was evaporated under reduced pressure to obtain 29.11 g of an oily product. A small amount of ethanol was added for crystallization, and the product was recrystallized to obtain 2-(3,4-dimethoxyphenyl)-2-isopropylacetonitrile.

Yield: 19.2 g (8.77 mmol), 63 %.

(b) Synthesis of 5-chloro-2-(3,4-dimethoxyphenyl)-2-isopropylvaleronitrile:

0.32 g (8.2 mmol) of sodium amide was put in a three-neck flask flushed with argon, and after further flushing with argon, 5 ml of 1,2-dimethoxyethane was added. 1.49 g (6.8 mmol) of 2-(3,4-dimethoxyphenyl)-2-isopropylacetonitrile was put in another flask, which was then flushed with argon, and then 10 ml of 1,2-dimethoxyethane was added to it. This was dropwise added to the above-prepared sodium amide suspension with a cylinder. After a while, the reaction mixture became white and cloudy. The mixture was drawn out with a syringe and added dropwise to 1,2-dimethoxyethane (50 ml) containing 2.5 ml (26.3 mmol) of 1-bromo-3-chloropropane which had been prepared in another argon-flushed flask, and then the whole was stirred overnight at room temperature. Water was added to the reaction mixture, which was then extracted with chloroform and dried with anhydrous magnesium sulfate. The solvent wes evaporated under reduced pressure to obtain 2.12 g of a crude product. Unreacted starting material was found still in the reaction mixture by TLC ($SiO_2$/$CHCl_3$), but the reaction mixture was used in the next reaction as such, without being further purified.

(c) Synthesis of 2-[3,4-dimethoxyphenyl]-5-[4-(4-fluorobenzoyl)1-piperidinyl]-2-isopropylvaleronitrile:

50 ml of toluene and 2.12 g of the crude 5-chloro-2 (3,4-dimethoxyphenyl)-2-isopropylvaleronitrile were added to a mixture comprising 2.6 g (10.7 mmol) of 4-(4-fluorobenzoyl)piperidine hydrochloride and 0.9 g (8.5 mmol) of anhydrous sodium carbonate, and heated under reflux. The reaction was followed by TLC and heating was stopped at an appropriate point. Water was added to the reaction mixture, which was then extracted with chloroform. After drying with anhydrous magnesium sulfate, the solvent was evaporated under reduced pressure, and the crude product thus obtained was purified by silica gel column chromatography ($CHCl_3$/MeOH) to obtain the above-titled compound.

Yield: 0.76 g (1.6 mmol). (0.2 g of the compound was converted into its hydrochloride).

TLC

($CHCl_3$ : MeOH = 9:1)

$R_f$ = 0.52

MS (FD, m/z)

466 ($M^+$)

$^1$H-NMR (TMS/$CDCl_3$, $\delta$/ppm)

0.80 (3H, d, J = 7Hz), 1.20 (3H, d, J = 7Hz) 1.9-3.5 (15H, m), 3.83 (3H, z), 3.88 (3H, z), 6.8-7.2 (5H, m), 7.8-8.1 (2H, m)

Example 6: Synthesis of 4-(4-fluorobenzoyl)-1-(4-phenylbutyl)-piperidine hydrochloride:

(a) Production of 4-phenylbutyl chloride:

2.2 ml (20 mmol) of thionyl chloride was added dropwise to a methylene chloride solution (30 ml) containing 3 g (20 mmol) of 4-phenyl-1-butanol, and stirred overnight at room temperature. The reaction solution was washed with water, aqueous 50 % sodium bicarbonate solution, water, 1H hydrochloric acid and water in that order, and then the solvent was dried with anhydrous magnesium sulfate. After distillation under reduced pressure, 3.62 g of a crude 4-phenylbutyl chloride was obtained. This was used in the next reaction without being further purified.

(b) Synthesis of 4-(4-fluorobenzoyl)-1-(4-phenylbutyl)piperidine:

A suitable amount of toluene, 1.8 g (12 mmol) of sodium iodide and 31 g of the crude phenylbutyl chloride were added to a mixture comprising 3 g (12.3 mmol) of 4-(4-fluorobenzoyl)piperidine hydrochloride and 2.6 g (24.5 mmol) of anhydrous sodium carbonate and heated under reflux. After reacting overnight, the heating was stopped. Water was added to the reaction mixture, which was then extracted with chloroform. The resulting extract was dried with anhydrous magnesium sulfate, and the solvent was evaporated under reduced pressure. The crude product thus obtained was purified by silica gel column chromatography to obtain the above-titled compound.

Yield: 0.13 g (0.38 mmol), 3 %.

TLC

($CHCl_3$ : MeOH = 5:1)

$R_f$ = 0.72

MS (FD, m/z)

339 ($M^+$)

$^1$H-NMR (TMS/$CDCl_3$, $\delta$/ppm)

1.4-2.2 (10 H,m), 2.2-2.7 (4H, m) 2.8-3.2 (3H, m) 6.9-7.2 (7H, m) 7.7-8.0 (2H, m)

Example 7: Synthesis of 1-[3-[2-cinnamoylaminophenylthio]-propyl]-4-(4-fluorobenzoyl)piperidine:

(a) Synthesis of 1-chloro-3-(2-aminophenylthio)propane:

20 ml of an isopropyl alcohol solution of 6.25 g (50.0 mmol) of 2-aminothiophenol was added to 20 ml of an isopropyl alcohol solution of 2.97 g (55.0 mmol) of sodium methoxide in a nitrogen stream atmosphere and blended. After being stirred for 30 minutes, the reaction mixture was cooled to 0 °C and 10 ml of a toluene solution of 1-bromo-3-chloropropane was added dropwise. After completion of the addition, the whole was stirred for 1 hour at room temperature and then heated under reflux for 1 hour. Then 60 ml of 1N sodium hydroxide was added and the reaction mixture was extracted with 50 ml of ether. The solvent was

evaporated to obtain a crude product of the above titled-compound.

Yield: 9.52 g (47.2 mmol), 94 %.

TLC

($Et_2O$)

$R_f$ = 0.85

$^1$H-NMR (TMS/$CDCl_3$, δ/ppm)

1.83 (2H, tt, J = 8, 8Hz), 2.80 (2H, t, J = 8 Hz), 3.57 (2H, t, J = 8Hz), 4.20 (2H, bz), 6.50 (1H, dd, J = 6.8 Hz) 6.62 (1H, d, J = 8 Hz), 7.05 (1H, dd, J = 8, 8 Hz), 7.26 (1H, d, J = 6 Hz)

(b) Synthesis of 1-chloro-3-(2-cinnamoylaminophenylthio)-propane:

10 ml of a dichloromethane solution of 3.33 g (20.0 mmol) of cinnamoyl chloride was added dropwise to 20 ml of a dichloromethane solution containing 4.02 g (20.0 mmol) of 1-chloro-3-(2-aminophenylthio)-propane and 2.02 g (20.0 mmol) of triethylamine at room temperature. After completion of the dropwise addition the whole was stirred overnight. After washing twice with 30 ml of 1N hydrochloric acid and then once with water, the organic layer was dried with anhydrous magnesium sulfate. The solvent was evaporated, and then the residue was recrystallized from dichloromethane-ether-hexane to obtain the above-titled compound.

Yield: 4.70 g (14.2 mmol), 71%.

TLC

($Et_2O$)

$R_f$ = 0.88

$^1$H-NMR (TMS/$CDCl_3$, δ/ppm)

2.00 (2H, tt, J = 8, 8 Hz), 2.88 (2H, t, J = 8 Hz), 3.57 (2H, t, J = 8 Hz), 6.50 (1H, d, J = 18 Hz), 6.85-7.55 (9H, m) 7.65 (1H, d, J = 18 Hz), 8.55 (1H, bz)

(c) Synthesis of 1-[3-(2-cinnamoylaminophenylthio)propyl]-4-(4-fluorobenzoyl)piperidine:

Using 3.17 g (9.58 mmol) of 1-chloro-3[2-(cinnamoylamino)phenylthio]propane, 2.33 g (9.58 mmol) of 4-(4-chloro-benzoyl)piperidine hydrochloride, 2.76 g (20.0 mmol) of potassium carbonate and 30 ml of isoamyl alcohol as starting materials, the same reaction was Example 1 was conducted. The product obtained was purified by column chromatography (eluent: chloroform/methanol = 40/1) to obtain the above-titled compound.

Yield 2.97 g (6.10 mmol), 64 %.

TLC

($CHCl_3$ : MeOH = 9 : 1)

$R_f$ = 0.51

MS (FD, m/z)

502 ($M^+$)

$^1$H-NMR (TMS/DMSO-$d_6$, δ/ppm)

1.30-2.02 (7H,m), 2.95-3.22 (8H, m) 7.10 (1H, d, J = 8, 8 Hz), 7.23 (1H, dd, J = 8, 8Hz), 7.31 (1H, dd, J = 8, 8 Hz), 7.39 (1H, dd, J = 9, 9 Hz), 7.41 (2H, dd, J = 8, 8 Hz), 7.46 (1H, d, J = 8 Hz), 7.55 (1H, d, J = 8 Hz), 7.60 (2H, d, J = 18 Hz), 7.65 (2H, d, J = 8 Hz), 7.71 (1H, d, J = 8 Hz), 8.06 (2H, dd, J = 8, 6Hz), 9.64 (1H, z)

Example 8: Synthesis of 1-[3-(2-aminophenylthio)-1-propyl]-4-(4-fluorobenzoyl)piperidine

Using 3.73 g (18.5 mmol) of 1-chloro-3-(2-aminophenylthio)propane, 4.52 g (18.5 mmol) of 4-(4-fluorobenzoyl)-piperidine hydrochloride, 6.90 g (50.0 mmol) of potassium carbonate and 50 ml of butanol as starting materials, the same reaction as Example 1 was conducted. The product obtained was purified by silica gel chromatography (eluent: chloroform/methanol = 40/1) to obtain the above-titled compound.

Yield: 0.50 g (1.12 mmol) in the form of dihydrochloride, 6 %.

TLC

($CHCl_3$ : MeOH = 9:1)

$R_f$ = 0.66

MS (FD, m/z)

373 ($M^+$)

$^1$H-NMR (TMS/DMSO-$d_6$ δ/ppm)

1.80-2.04 (7H,m), 2.99 (2H, t, J = 7Hz) 3.02-3.12 (2H,m), 3.20 (2H, t, J = 8Hz) 3.52 (2H, d, J = 12Hz), 4.56 (2H, bz) 7.00-7.12 (1H,m), 7.21-7.32 (2H,m) 7.39 (2H, dd, J = 10, 10Hz), 7.55 (1H, d, J = 8 Hz), 8.10 (2H, dd, J = 8, 6 Hz)

Example 9: Synthesis of 4-(4-fluorobenzoyl)-1-(5-phenylpentyl)piperidine hydrochloride:

(a) Synthesis of 5-phenylpentyl bromide:

1.8 g (6.7 mmol) of phosphorus tribromide was added to a chloroform solution (20 ml) of 3.3 g (20 mmol) of 5-phenyl-1-pentanol and heated under reflux for 4 hours. When the reaction was followed by TLC, the starting materials were found still the reaction mixture, and so 0.2 g of phosphorus tribromide was further added and heated under reflux. Afterwards, the reaction solution was washed with water and dried with anhydrous magnesium sulfate, and then the solvent was evaporated under reduced pressure. Thus, 4.30 g of crude 5-phenylpentyl bromide was obtained.

(b) Synthesis of 4-(4-fluorobenzoyl)-1-(5-phenylpentyl)-piperidine:

50 ml of isopropyl alcohol and 2.26 g of the crude 5-phenylpentyl bromide were added to a mixture comprising 2.4 g (9.9 mmol) of 4-(4-fluorobenzoyl)piperidine hydrochloride and 2.1 g (19.8 mmol) of anhydrous sodium carbonate, and heated under reflux. The reaction was continued overnight and then the heating was stopped. The reaction mixture was concentrated under reduced pressure, and water was added thereto. This was extracted with chloroform. The resulting extract was dried with anhydrous magnesium sulfate and then the solvent was evaporated under reduced pressure. The crude product thus obtained was purified by silica gel column chromatography to obtain the above-titled compound.

Yield: 2.18 g, 62 %.

TLC

($CHCl_3$ : MeOH = 9:1)

$R_f$ - 0.41

MS (FD, m/z)

353 ($M^+$)

The compounds cited in Examples 10 to 108 were synthesised by the same procedure described above.

exp 1Ø

2-(3,4-Dimethoxyphenyl)-5-[4-(4-fluorobenzoyl)-1-piperidinyl]valeronitrile L-(+)-tartarate
TLC(CHCL3:MeOH=9:1)
Rf=Ø.48
MS(FD,m/z)
424(M+)
H-NMR(TMS/CDCL3, δ/ppm)
1.8-2.4(8H,m) 2.9-3.7(6H,m)
3.85(3H,s) 3.88(3H,s)
3.8-4.4(2H,m) 6.8-6.9(3H,m)
7.1-7.2(2H,m) 7.9-8.Ø(2H,m)

exp11

5-[4-(4-Fluorobenzoyl)-1-piperidinyl]-2-isopropyl-2-(3,4,5-trimethoxyphenyl)valeronitrile L-(+)-tartarate
TLC(CHCL3:MeOH=9:1)
Rf=Ø.48
MS(FD,m/z)
497(M+)
H-NMR(TMS/CDCL3, δ/ppm)
Ø.76(3H,bs) 1.Ø-1.2(1H,m)
1.16(3H,bs) 1.4-2.5(12H,m)
2.9-3.4(3H,m) 3.82(3H,s)
3.87(3H,s) 6.6-6.8(2H,m)

6.6-6.8(2H,m) 7.∅-7.2(2H,m)
7.8-8.1(2H,m)

exp 12

2-(3,4-Dimethoxyphenyl)-2-dodecyl-5-[4-(4-fluorobenzoyl)-1-piperidinyl]valeronitrile hydrochloride
TLC(CHCL3:MeOH = 9:1)
Rf = ∅.70
MS(FD,m/z)
592(M + )
H-NMR(TMS/DMSO-d6, δ/ppm)
∅.84(3H,t,J = 8Hz) 1.2-1.5(22H,m)
1.8-2.1(8H,m) 2.9-3.1(4H,m)
3.4-3.5(2H,m) 3.6-3.8(1H,m)
3.76(3H,s) 3.79(3H,s)
6.9-7.∅(3H,m) 7.4∅(2H,dd,J = 8,8Hz)
8.∅7(2H,dd,J = 1∅,8Hz)

exp 13

2-(3,4-Dimethoxyphenyl)-2-{3-[4-(4-fluorobenzoyl)-1-piperidinyl]-1-propyl}-1-3-dithiane-1,1,3,3-tetraoxide
TLC(CHCL3:MeOH = 5:1)
Rf = ∅.58
MS(FD,m/z)
567(M + )
H-NMR(TMS/CDCL3, δ/ppm)
2.∅-2.1(4H,m) 2.5-4.∅(17H,m)
3.9∅(6H,s) 4.45(3H,t,J = 1∅Hz)
6.91(1H,d,J = 8Hz) 7.18(2H,dd,J = 8,8Hz)
7.46(1H,dd,J = 8,2Hz) 7.67(1H,d,J = 2Hz)
7.93(2H,dd,J = 1∅,8Hz)

exp 14

5-[4-(3,4-Dimethoxybenzoyl)-1-piperidinyl]-2-(3,4-dimethoxyphenyl)-2-isopropylvaleronitrile hydrochloride
TLC(CHCL3:MeOH = 9:1)
Rf = ∅.83
MS(FD,m/z)
5∅8(M + )
H-NMR(TMS/CDCL3, δ/ppm)
∅.8∅(3H,t,J = 6Hz) 1.21(3H,t,J = 6Hz)
2.∅-3.8(16H,m) 3.89(3H,s)
3.93(3H,s) 3.96(6H,s)
6.86(1H,d,J = 8Hz) 6.91(1H,d,J = 8Hz)
6.94(1H,d,J = 2Hz) 7.∅∅(1H,dd,J = 8,2Hz)
7.44(1H,d,J = 2Hz) 7.49(1H,dd,J = 8,2Hz)

exp 15

2-(3,4-Dichlorophenyl)-5-[4-(4-fluorobenzoyl)-1-piperidinyl]-2-isopropylvaleronitrile hydrochloride
TLC(CHCL3:MeOH = 9:1)
Rf = ∅.88
MS(FD,m/z)
474(M + )
H-NMR(TMS/CDCL3, δ/ppm)
∅.8∅(3H,d,J = 8Hz) 1.22(3H,d,J = 8Hz)
1.8-3.8(16H,m) 7.18(2H,dd,J = 8,8Hz)
7.32(1H,dd,J = 2,8Hz) 7.52(1H,d,J = 8Hz)

7.58(1H,d,J = 2Hz) 7.92(2H,dd,J = 1Ø,8Hz)

exp 16

2-(3-Benzoylphenyl)-5-[4-(4-fluorobenzoyl)-1-piperidinyl]-2-isopropyl valeronitrile L-( + )-tartarate
TLC(CHCL3:MeOH = 9:1)
Rf = Ø.56
MS(FD,m/z)
482(M + )
H-NMR(TMS/CDCL3, δ/ppm)
1.4-1.6(1H,m) 1.6-1.9(5H,m)
1.77(3H,s) 2.Ø-2.2(4H,m)
2.37(2H,pseud t,J = 7Hz) 2.8-3.Ø(2H,m)
3.3-3.6(2H,m) 4.45(1H,bs)
6.9-7.4(9H,m)

exp 17

2-(3,4-Dimethoxyphenyl)-5-[4-(α-hydroxy-4-fluorobenzyl)-1-piperidinyl]-2-isopropylvaleronitrile L-( + )-tartarate
TLC(CHCL3:MeOH = 9:1)
Rf = Ø.27
MS(FD,m/z)
469(MH + )
H-NMR(TMS/CDCL3, δ/ppm)
Ø.78(3H,d,J = 7Hz) 1.1-1.2(4H,m)
1.16(3H,d,J = 7Hz) 1.2-1.4(1H,m)
1.4-1.6(2H,m) 1.6-1.9(4H,m)
2.Ø-2.1(2H,m) 2.19(2H,pseud t,J = 8Hz)
2.6-2.9(2H,m) 3.86(3H,s)
3:87(3H,s) 4.26(1H,d,J = 7Hz)
6.8-7.1(5H,m) 7.1-7.3(2H,m)

exp 18

1-(4-Phenylbutyl)-α-(4-fluorophenyl)-4-piperidinemethanol L-( + )-tartarate
TLC(CHCL3:MeOH = 9:1)
Rf = Ø.21
MS(FD,m/z)
341(M + )
H-NMR(TMS/CDCL3, δ/ppm)
1.3-2.2(1ØH,m) 2.4-2.7(4H,m)
2.8-3.Ø(2H,m) 3.3-3.6(2H,m)
4.45(1H,bs) 6.9-7.4(9H,m)

exp 19

2,2-Diphenyl-5-[4-(4-fluorobenzoyl)-1-piperidinyl]valeronitrile hydrochloride
TLC(CHCL3:MeOH = 9:1)
Rf = Ø.6Ø
MS(FD,m/z)
448(M + )
H-NMR(TMS/CDCL3, δ/ppm)
1.9-2.1(4H,m) 3.Ø-3.1(4H,m)
3.2-3.3(2H,m) 3.7-3.8(1H,m)
7.18(2H,dd,J = 8,8Hz) 7.2-7.5(1ØH,m)
7.92(2H,dd,J = 1Ø,8Hz)

exp 20

5-{4-[Bis(4-fluorophenyl)methrlene]-1-piperidinyl}-2-(3,4-dimethoxyphenyl)-2-isopropylvaleronitrile hydrochloride
TLC(CHCL3:MeOH = 9:1)
Rf = ∅.54
MS(FD,m/z)
544(M + )
H-NMR(TMS/CDCL3, δ/ppm)
∅.78(3H,d,J = 8Hz) 1.2∅(3H,d,J = 8Hz)
1.6-3.6(15H,m) 3.87(3H,s)
3.94(3H,s) 6.8-7.1(11H,m)

exp 21

2-(3,4-Dimethoxyphenyl)-6-[4-(4-fluorobenzoyl)-1-piperidinyl]-2-isopropylhexanenitrile hydrochloride
TLC(CHCL3:MeOH = 9:1)
Rf = ∅.54
MS(FD,m/z)
48∅(M + )
H-NMR(TMS/CDCL3, δ/ppm)
∅.8∅(3H,d,J = 7Hz) ∅.9-1.1(1H,m)
1.18(3H,d,J = 7Hz) 1.3-1.7(3H,m)
1.8-1.9(5H,m) 2.∅-2.2(4H,m)
2.3∅(2H,pseud t) 2.9-3.∅(2H,m)
3.2∅(1H,pseud hept) 3.89(3H,s)
3.9∅(3H,s) 6.8-6.9(3H,m)
7.∅-7.2(2H,m) 7.9-8.∅(2H,m)

exp 22

2-(3,4-Dimethoxyphenyl)-2-isopropyl-5-[4-(2,4,6-trimethylbenzoyl)-1-piperidinyl]valeronitrile hydrochloride
TLC(CHCL3:MeOH = 9:1)
Rf = ∅.64
MS(FD,m/z)
49∅(M + )
H-NMR(TMS/CDCL3, δ/ppm)
∅.8-1.3(6H,m) 1.9-3.6(25h,m)
3.9-4.∅(6H,m) 6.8-7.∅(5H,m)

exp 23

2-Dodecyl-5-[4-(4-fluorobenzoyl)-1-piperidinyl]-2-(3-methoxyphenyl)valeronitrile hydrochloride
TLC(CHCL3:MeOH = 9:1)
Rf = ∅.65
MS(FD,m/z)
562(M + )
H-NMR(TMS/CDCL3, δ/ppm)
∅.88(3H,t,J = 8Hz) 1.2-3.8(37H,m)
3.88(3H,s) 6.8-7.∅(3H,m)
7.27(2H,dd,J = 8,8Hz) 7.32(1H,dd,J = 8,8Hz)
7.92(2H,dd,J = 1∅,8Hz)

exp 24

5-[4-(4-Fluorobenzoyl)-1-piperidinyl]-2-phenylvaleronitrile hydrochloride
TLC(CHCL3:MeOH = 9:1)
Rf = ∅.54

MS(FD,m/z)
364(M+)
H-NMR(TMS/CDCL3, δ/ppm)
1.6-1.8(2H,m) 1.8-1.9(4H,m)
1.9-2.∅(2H,m) 2.∅-2.2(2H,m)
2.41(2H,t,J=7Hz) 2.9-3.∅(2H,m)
3.2∅(1H.m) 3.88(1H,t,J=7Hz)
7.1-7.2(2H,m) 7.3-7.4(5H,m)
7.9-8.∅(2H.m)

exp 25

2-(3-Chloropropyl)-5-[4-(4-fluorobenzoyl)-1-piperidinyl]-2-phenylvaleronitrile hydrochloride
TLC(CHCL3:MeOH=9:1)
Rf=∅.61
MS(FD,m/z)
44∅(M+)
H-NMR(TMS/CDCL3, δ/ppm)
1.2-1.4(1H,m) 1.5-1.9(4H,m)
1.9-2.2(7H,m) 2.31(2H,t,J=8Hz)
2.8-3.∅(2H,m) 3.18(1H.m)
3.46(2H,t,J=6Hz) 7.∅-7.2(2H,m)
7.3-7.5(5H,m) 7.9-8.∅(2H.m)

exp 26

2-(3,4-Dimethoxyphenyl)-7-[4-(4-fluorobenzoyl)-1-piperidinyl]-2-isopropylheptanenitrile hydrochloride
TLC(CHCL3:MeOH=9:1)
Rf=∅.54
MS(FD,m/z)
494(M+)
H-NMR(TMS/CDCL3, δ/ppm)
∅.8∅(3H,d,J=7Hz) 1.∅3(1H,m)
1.19(3H,d,J=7Hz) 1.2-1.5(5H,m)
1.7-1.9(5H,m) 2.∅-2.2(4H,m)
2.28(2H,t) 2.9-3.∅(2H,m)
3.2∅(1H,pseud quin) 3.88(3H,s)
3.9∅(3H,s) 6.8-7.∅(3H,m)
7.1-7.2(2H,m) 7.9-8.∅(2H,m)

exp 27

2-(3,4-Dimethoxyphenyl)-5-[4-(4-fluorobenzoyl)-1-piperidinyl]-2-phenylthiovaleronitrile hydrochloride
TLC(CHCL3:MeOH=9:1)
Rf=∅.69
MS(FD,m/z)
532(M+)
H-NMR(TMS/CDCL3, δ/ppm)
2.∅-3.8(15H,m) 3.88(3H,S)
3.9∅(3H,s) 6.8-7.∅(3H,m)
7.18(2H,dd,J=8,8Hz) 7.3-7.5(5H,m)
7.92(2H,dd,J=1∅,8Hz)

exp 28

5-[4-(4-Fluorobenzoyl)-1-piperidinyl]-2-isopropyl-2-(1-naphthyl)valeronitrile hydrochloride
TLC(CHCL3:MeOH=9:1)
Rf=∅.68

MS(FD,m/z)
456(M+)
H-NMR(TMS/CDCL3, δ/ppm)
Ø.82(3H,bs) 1.35(3H,bs)
1.8-3.7(16H,m) 7.16(2H,dd,J=8,8Hz)
7.2-7.9(9H,m)

exp 29

5-[4-(4-fluorobenzoyl)-1-piperidinyl]-2-isopropyl-2-(2-naphthyl)valeronitrile hydrochloride
TLC(CHCL3:MeOH=9:1)
Rf=Ø.65
MS(FD,m/z)
456(M+)
H-NMR(TMS/CDCL3, δ/ppm)
Ø.8Ø(3H,d,J=6Hz) 1.28(3H,d,J=6Hz)
1.5-3.7(16H,m) 7.1-8.Ø(11H,m)

exp 30

2-(3,4-Dimethoxyphenyl)-5-[4-(4-fluorophenyl)methylene-1-piperidinyl]-2-isopropylvaleronitrile hydrochloride
TLC(CHCL3:MeOH=9:1)
Rf=Ø.73
MS(FD,m/z)
45Ø(M+)
H-NMR(TMS/CDCL3, δ/ppm)
Ø.8Ø(3H,d,J=8Hz) 1.2Ø(3H,d,J=8Hz)
1.5-3.6(15H,m) 3.9-4.Ø(6H,m)
6.4-6.5(1H,m) 6.8-7.2(7H,m)

exp 31

5-[4-(4-Fluorobenzoyl)-1-piperidinyl]-2-isopropyl-2-(3-trifluoromethylphenyl)valeronitrile hydrochloride
TLC(CHCL3:MeOH=9:1)
Rf=Ø.64
MS(FD,m/z)
474(M+)
H-NMR(TMS/CDCL3, δ/ppm)
Ø.8Ø(3H,d,J=8Hz) 1.23(3H,d,J=8Hz)
1.7-1.8(1H,m) 2.Ø-3.4(14H,m)
3.7-3.8(1H,m) 7.18(2H,dd,J=8,8Hz)
7.6-7.7(3H,m) 7.92(2H,dd,J=1Ø,8Hz)

exp 32

2-(3,4-Dimethoxyphenyl)-8-[4-(4-Fluorobenzoyl)-1-piperidinyl-2-isopropyloctanenitrile hydrochloride
TLC(CHCL3:MeOH=9:1)
Rf=Ø.53
MS(FD,m/z)
5Ø8(M+)
H-NMR(TMS/CDCL3, δ/ppm)
Ø.8Ø(3H,d,J=7Hz) Ø.9-1.1(1H,m)
1.19(3H,d,J=7Hz) 1.2-1.5(1ØH,m)
1.7-1.9(5H,m) 2.Ø-2.2(4H,m)
2.33(2H,pseud t) 2.9-3.Ø(2H,m)
3.2(1H,pseud quin) 3.89(3H,s)
3.9Ø(3H,s) 6.8-7.Ø(3H,m)
7.1-7.2(2H,m) 7.9-8.Ø(2H,m)

exp 33

5,6-Dimethoxy-1-{3-[4-(4-fluorobenzoyl)-1-piperidinyl]propyl}-1-indanenitrile hydrochloride
TLC(CHCL3:MeOH = 9:1)
Rf = ∅.74
MS(FD,m/z)
45∅(M + )
H-NMR(TMS/CDCL3, δ/ppm)
1.8-3.5(18H,m) 3.7-3.8(1H,m)
3.88(3H,s) 3.92(3H,s)
6.78(1H,s) 6.85(1H,s)
7.19(2H,dd,J = 8,8Hz) 7.94(2H,dd,J = 1∅,8Hz)

exp 34

2-(3,4-Dihydroxyphenyl)-2-dodecyl-5-[4-(4-fluorobenzoyl)-1-piperidinyl]valeronitrile hydrochloride
TLC(CHCL3:MeOH = 9:1)
Rf = ∅.57
MS(FD,m/z)
565(MH + )
H-NMR(TMS/CDCL3, δ/ppm)
∅.88(3H,d,J = 6Hz) 1.1-3.8(39H,m)
6.8-7.2(5H,m) 7.9-8.∅(2H,m)

exp 35

2-(3,4-Dimethoxyphenyl)-2-dodecyl-5-[4-(α-hydroxy-4-fluorobenzyl)-1-piperidinyl]valeronitrile hydrochloride
TLC(CHCL3:MeOH = 9:1)
Rf = ∅.39
MS(FD,m/z)
565(M + )
H-NMR(TMS/CDCL3, δ/ppm)
∅.88(3H,d,J = 6Hz) 1.1-1.3(22H,m)
1.8-3.6(16H,m) 3.8-3.9(6H,m)
4.41(1H,d,J = 8Hz) 6.8-7.3(7H,m)

exp 36

5-[4-(4-Fluorobenzoyl)-1-piperidinyl]-2-(1-methylpyrrole-2-yl)valeronitrile hydrochloride
TLC(CHCL3:MeOH = 9:1)
Rf = ∅.42
MS(FD,m/z)
367(M + )
H-NMR(TMS/CDCL3, δ/ppm)
1.7-1.9(6H,m) 1.9-2.2(4H,m)
2.4-2.5(2H,m) 2.9-3.1(2H,m)
3.21(1H,hep) 3.65(3H,s)
3.97(1H,dd,J = 7,9Hz) 6.∅6-6.∅9(1H,m)
6.1∅-6.13(1H,m) 6.59-6.61(1H,m)
7.1∅-7.17(2H,m)
7.93-7.99(2H,m)

exp 37

2-Butyl-2-(3,4-dimethoxyphenyl)-5-[4-(4-fluorobenzoyl)-1-piperidinyl]valeronitrile hydrochloride
TLC(CHCL3:MeOH = 9:1)
Rf = ∅.67
MS(FD,m/z)

480(M+)
H-NMR(TMS/CDCL3, δ/ppm)
0.86(3H,d,J=8Hz) 1.0-3.8(21H,m)
3.88(3H,s) 3.96(3H,s)
6.87(1H,d,J=8Hz) 6.92(1H,s)
6.99(1H,d,J=8Hz) 7.18(2H,dd,J=8,8Hz)
7.92(2H,dd,J=10,8Hz)

exp 38

2-(3,4-Dimethoxyphenyl)-5-[4-(4-fluorobenzoyl)-1-piperidinyl]-2-octylvaleronitrile hydrochloride
TLC(CHCL3:MeOH=9:1)
Rf=0.70
MS(FD,m/z)
536(M+)
H-NMR(TMS/CDCL3, δ/ppm)
0.86(3H,d,J=8Hz) 1.0-3.8(14H,m)
3.88(3H,s) 3.96(3H,s)
6.87(1H,d,J=8Hz) 6.92(1H,d,J=2Hz)
6.99(1H,dd,J=8,2Hz) 7.18(2H,dd,J=8,8Hz)
7.90(2H,dd,J=10,8Hz)

exp 39

5-[4-(4-Fluorobenzoyl)-1-piperidinyl]-2-isopropyl-2-(1-methylpyrrole-2-yl)valeronitrile hydrochloride
TLC(CHCL3:MeOH=9:1)
Rf=0.54
MS(FD,m/z)
409(M+)
H-NMR(TMS/CDCL3, δ/ppm)
1.00(3H,d,J=7Hz) 1.08(3H,d,J=7Hz)
1.4-1.5(1H,m) 1.5-1.7(1H,m)
1.7-1.9(4H,m) 1.9-2.1(4H,m)
2.24(1H,hept,J=7Hz) 2.3-2.4(2H,m)
2.8-2.9(2H,m) 3.18(1H,hep)
3.74(3H,s) 6.00-6.03(1H,m)
6.10-6.14(1H,m) 6.52-6.54(1H,m)
7.09-7.16(2H,m) 7.93-7.98(2H,m)

exp 40

2-(3,4-Dimethoxyphenyl)-5-[4-(4-fluorobenzoyl)-1-piperidinyl]-2-hexadecylvaleronitrile hydrochloride
TLC(CHCL3:MeOH=9:1)
Rf=0.76
MS(FD,m/z)
648(M+)
H-NMR(TMS/CDCL3,δ/ppm)
0.88(3H,t,J=8Hz) 1.0-3.8(45H,m)
3.88(3H,s) 3.96(3H,s)
6.86(1H,d,J=8Hz) 6.92(1H,s)
6.99(1H,d,J=8Hz) 7.18(2H,dd,J=8,8Hz)
7.91(2H,dd,J=10,8Hz)

exp 41

2-(3,4-Dimethoxyphenyl)-5-[4-(4-fluorobenzoyl)-1-piperidinyl]-2-methylvaleronitrile

exp 42

2-(3,4-Dimethoxyphenyl)-2-ethyl]-5-[4-(4-fluorobenzoyl)-1-piperidinyl]valeronitrile

exp 43

2-(3,4-Dimethoxyphenyl)-5-[4-(4-fluorobenzoyl)-1-piperidinyl]-2-propylvaleronitrile

exp 44

2-(3,4-Dimethoxyphenyl)-5-[4-(4-fluorobenzoyl)-1-piperidinyl]-2-pentylvaleronitrile

exp 45

2-(3,4-Dimethoxyphenyl)-5-[4-(4-fluorobenzoyl)-1-piperidinyl]-2-hexylvaleronitrile

exp 46

2-(3,4-Dimethoxyphenyl)-5-[4-(4-fluorobenzoyl)-1-piperidinyl]-2-propylvaleronitrile

exp 47

2-(3,4-Dimethoxyphenyl)-5-[4-(4-fluorobenzoyl)-1-piperidinyl]-2-nonylvaleronitrile

exp 48

2-Decl-2-(3,4-dimethoxyphenyl)-5-[4-(4-fluorobenzoyl)-1-piperidinyl]valeronitrile

exp 49

2-(3,4-Dimethoxyphenyl)-5-[4-(4-fluorobenzoyl)-1-piperidinyl]-2-undecylvaleronitrile

exp 50

2-(3,4-Dimethoxyphenyl)-5-[4-(4-fluorobenzoyl)-1-piperidinyl]-2-tridecylvaleronitrile

exp 51

2-(3,4-Dimethoxyphenyl)-5-[4-(4-fluorobenzoyl)-1-piperidinyl]-2-tetradecylvaleronitrile

exp 52

2-(3,4-Dimethoxyphenyl)-5-[4-(4-fluorobenzoyl)-1-piperidinyl]-2-pentadecylvaleronitrile

exp 53

2-Allyl-2-(3,4-dimethoxyphenyl)-5-[4-(4-fluorobenzoyl)-1-piperidinyl]valeronitrile

exp 54

2-Cyclopropyl-2-(3,4-dimethoxyphenyl)-5-[4-(4-fluorobenzoyl)-1-piperidinyl]valeronitrile

exp 55

2-Cyclobutyl-2-(3-4-dimethoxyphenyl)-5-[4-(4-fluorobenzoyl)-1-piperidinyl]valeronitrile

exp 56

2-Cyclopentyl-2-(3-4-dimethoxyphenyl)-5-[4-(4-fluorobenzoyl)-1-piperidinyl]valeronitrile

exp 57

2-Cyclohexyl-2-(3-4-dimethoxyphenyl)-5-[4-(4-fluorobenzoyl)-1-piperidinyl]valeronitrile

exp 58

2-Cyclooctyl-2-(3-4-dimethoxyphenyl)-5-[4-(4-fluorobenzoyl)-1-piperidinyl]valeronitrile

exp 59

2-Benzyl-2-(3-4-dimethoxyphenyl)-5-[4-(4-fluorobenzoyl)-1-piperidinyl]valeronitrile

exp 60

2-(3-4-Dimethoxyphenyl)-4-[4-(4-fluorobenzoyl)-1-piperidinyl]-2-dodecylbutyronitrile

exp 61

2-(3-4-Dimethoxyphenyl)-6-[4-(4-fluorobenzoyl)-1-piperidinyl]-2-dodecylhexanenitrile

exp 62

2-(3-4-Dimethoxyphenyl)-7-[4-(4-fluorobenzoyl)-1-piperidinyl]-2-dodecylheptanenitrile

exp 63

2-(3-4-Dimethoxyphenyl)-8-[4-(4-fluorobenzoyl)-1-piperidinyl]-2-dodecyloctanenitrile

exp 64

5-{4-[Bis(4-flurophenyl)methylene]-1-piperidinyl}-2-(3,4-dimethoxyphenyl)-2-dodecylvaleronitrile

exp 65

2-(3-4-Dimethoxyphenyl)-5-[4-(4-fluorobenzyl)-1-piperidinyl]-2-dodecylvaleronitrile

exp 66

5-(4-Benzyl-1-piperidinyl)-2-(3,4-dimethoxyphenyl)-2-dodecylvalenitrile

exp 67

5-(4-Benzoyl-1-piperidinyl)-2-(3,4-dimethoxyphenyl)-2-dodecylvalenitrile

exp 68

2-(3,4-Dimethoxyphenyl)-5-[4-(4-fluorobenzoyl)-1-piperidinyl]-2-dodecylvaleronitrile

exp 69

5-[4-(3,4-Dimethoxyphenyl)-1-piperidinyl]-2-(3,4-dimethoxyphenyl)-2-dodecylvaleronitrile

exp 70

2-(3,4-Dichlorophenyl)-2-dodecyl-5-[4-(4-fluorobenzoyl)-1-piperidinyl]valeronitrile

exp 71

2-(3,4-Dimethoxyphenyl)-2-dodecyl-5-[4-(2,4,6-trimethylbenzoyl)-1-piperidinyl]valeronitrile

exp 72

5-[4-(4-Fluorobenzoyl)-1-piperidinyl]-2-dodecyl-2-(1-naphthyl)valeronitrile

exp 73

5-[4-(4-Fluorobenzoyl)-1-piperidinyl]-2-dodecyl-2-(2-naphthyl)valeronitrile

exp 74

2-(3,4-Dimethoxyphenyl)-2-dodecyl-5-[4-(4-fluorophenyl)methylene-1-piperidinyl]valeronitrile

exp 75

5-[4-(4-Fluorobenzoyl)-1-piperidinyl]-2-dodecyl-2-(3-trifluoromethylphenyl)valeronitrile

exp 76

5-[4-(4-Fluorobenzoyl)-1-piperidinyl]-2-dodecyl-2-(1-methylpyrrole-2-yl) valeronitrile

exp 77

2-(3,4-Dimethoxyphenyl)-5-[4-(α-hydroxy-4-fluorobenzyl)-1-piperidinyl]-2-methylvaleronitrile

exp 78

2-(3,4-Dimethoxyphenyl)-2-ethyl-5-[4-(α-hydroxy-4-fluorobenzyl)-1-piperidinyl]valeronitrile

exp 79

2-(3,4-Dimethoxyphenyl)-5-[4-(α-hydroxy-4-fluorobenzyl)-1-piperidinyl]-2-propylvaleronitrile

exp 80

2-Butyl-2-(3,4-dimethoxyphenyl)-5-[4-(α-hydroxy-4-fluorobenzyl)-1-piperidinyl]valeronitrile

exp 81

2-(3,4-Dimethoxyphenyl)-5-[4-(α-hydroxy-4-fluorobenzyl)-1-piperidinyl]-2-pentylvaleronitrile

exp 82

2-(3,4-Dimethoxyphenyl)-2-hexyl-5-[4-(α-hydroxy-4-fluorobenzyl)-1-piperidinyl]valeronitrile

exp 83

2-(3,4-Dimethoxyphenyl)-2-heptyl-5-[4-(α-hydroxy-4-fluorobenzyl)-1-piperidinyl]valeronitrile

exp 84

2-(3,4-Dimethoxyphenyl)-5-[4-(α-hydroxy-4-fluorobenzyl)-1-piperidinyl]-2-octylvaleronitrile

exp 85

2-(3,4-Dimethoxyphenyl)-5-[4-(α-hydroxy-4-fluorobenzyl)-1-piperidinyl]-2-nonylvaleronitrile

exp 86

2-Decyl-2-(3,4-dimethoxyphenyl)-5-[4-(α-hydroxy-4-fluorobenzyl)-1-piperidinyl]valeronitrile

exp 87

2-(3,4-Dimethoxyphenyl)-5-[4-(α-hydroxy-4-fluorobenzyl)-1-piperidinyl]-2-undecylvaleronitrile

exp 88

2-(3,4-Dimethoxyphenyl)-5-[4-(α-hydroxy-4-fluorobenzyl)-1-piperidinyl]undecylvaleronitrile

exp 89

2-(3,4-Dimethoxyphenyl)-4-[4-(α-hydroxy-4-fluorobenzyl)-1-piperidinyl]butyronitrile

exp 90

2-(3,4-Dimethoxyphenyl)-6-[4-(α-hydroxy-4-fluorobenzyl)-1-piperidinyl]hexanenitrile

exp 91

2-(3,4-Dimethoxyphenyl)-7-[4-(α-hydroxy-4-fluorobenzyl)-1-piperidinyl]heptanenitrile

exp 92

2-(3,4-Dimethoxyphenyl)-8-[4-(α-hydroxy-4-fluorobenzyl)-1-piperidinyl]octanenitrile

exp 93

5-{3-[4-(4-Fluorobenzoyl)-1-piperidinyl]propyl}-2,3-dihydro-3-hydroxy-2--phenyl-1,5-benzothiazpine-4(5H)-one L-(+)-tartarate

exp 94

5-{3-[4-(4-Fluorobenzoyl)-1-piperidinyl]propyl}-2,3-dihydro-3-hydroxy-2--phenyl-1,5-benzothiazpine-4(5H)-one L-(+)-tartarate

exp 95

4-[4-(4-Fluorobenzoyl)-1-piperidinyl]-(3',4'-dimethoxyphenyl)butyrophenone

exp 96

4-[4-(5H-Dibenzo[a,d]cyclohepten-5-ylidene)-1-piperidinyl]- 4'-fluorobutyrophenone

exp 97

2-(3,4-Dimethoxyphenyl)-2-isopropyl-5-(4-phenyl-1-piperidinyl)valeronitrile

exp 98

2,2-Bis{3-[4-(4-fluorobenzoyl)-1-piperidinyl]-1-propyl}-(3-methoxyphenyl)acetonitrile

exp 99

2-{3-[N-(3,4-Dimethoxyphenyl)-N-methylamino]-1-propyl}-2-(3,4-dimethoxyphenyl)-5-[4-(fluorobenzoyl)-1-piperidinyl]valeronitrile

exp 100

3-{2-[4-(4-Fluorobenzoyl)-1-piperidinyl]-1-ethyl]-5-(2-methoxy-1-ethyl)-2,6-dimethyl-4-(3-nitrophenyl)-1,4-dihydropyridine-3,5-dicarboxylate hydrochloride

Example 109: Valuation of serotonin-antagonistic activity:

SD male rats (from 8th to 12th week-age, weight: about 300 g) were killed and the abdomen was cut to take out the aorta. This was cut into 2.5 pieces and hung in a Magnus tube kept at 37 ± 1 °C and containing 20 ml of a K.S. Linger solution, into which amixed gas of 95 % $o_2$ + 5 % $CO_2$ was introduced. These specimens were connected to an isotonic transducer and recorded under load of 1 g. The concentration of serotonin was from 1 x $10^{-7}$ M to 1 x $10^{-4}$ M; and the concentration of the product to be tested was from 1 x $10^{-4}$ M to 1 x $10^{-3}$ M. From the serotonin dose-reaction curve, $pA_2$ was calculated by the method of Van Rossem et al.

The affinity for the 5-$HT_2$ receptor was evaluated by a receptor binding assay based on the microsomal fraction of bovine cortex.

The results are summarised in Table 1.

Table 1

| Example No. | Serotonin-antagonistic Activity (*$pA_2$, pKi) |
|---|---|
| 3 | 8.3 * |
| 4 | 8.2 * |
| 5 | 8.2 * |
| 6 | 8.5 * |

| | |
|---|---|
| 7 | 8.5 * |
| 8 | 8.0 * |
| 9 | 8.0 * |
| 10 | 7.3 * |
| 11 | 7.7 * |
| 20 | 7.6 * |
| 25 | 8.9 |
| 26 | 8.9 |
| 38 | 7.8 |

Example 110: Evaluation of Antihypertensive Activity:

Six male SHR rats which had been bred under sufficient acclimatisationn and were ascertained to be hypertensive* (spontaneous hypertensive rats, weight: 400-440 g) were used as test animals.

1 ml of a physiological salt solution containing the product to be tested (10 mg/kg) in 2.5 % of Niocol and 2.5 % of ethanol was intravenously injected once. The blood pressure of the test animal was measured by closed (bloodless) blood pressure measuring method.

The results are summarised in Table 2.

Table 2

| Example No. | Time After Injection (hr) | Blood (mmHg) | Pressure $N/cm^2$ |
|---|---|---|---|
| 5 | 0.5 | -91 | -1.21 |
| 6 | 0.5 | -84 | -1.12 |
| 8 | 0.5 | -84 | -1.12 |
| 9 | 0.5 | -89 | -1.18 |
| 10 | 0.5 | -89 | -1.18 |
| 11 | 0.5 | -91 | -1.21 |
| 12 | 0.5 | -94 | -1.25 |
| 15 | 0.5 | -102 | -1.36 |
| 17 | 0.5 | -65 | -0.86 |
| 22 | 0.5 | -71 | -0.94 |
| 35 | 0.5 | -69 | -0.92 |
| 36 | 0.5 | -118 | -1.57 |
| 38 | 0.5 | -82 | -1.09 |
| 39 | 0.5 | -117 | -1.56 |

From the above results, it will be apparent that the ethylamine derivative of the present invention have both serotonin-antagonist activity and the antihypertensive activity and can therefore be used as an antihypertensive agent.

**Claims**

**1.** Use in the preparation of an antihypertensive medicament of an ethylamine derivative of the formula (I) or a salt thereof;

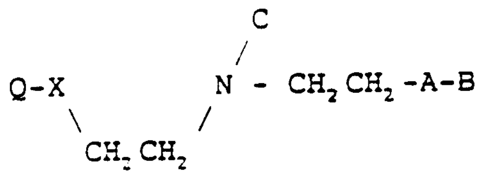

(I)

wherein the structure of the combined group -A-B is selected from the following:

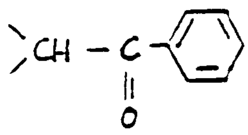

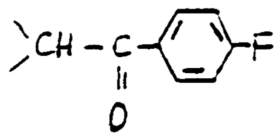

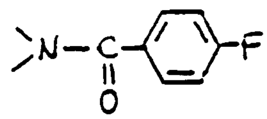

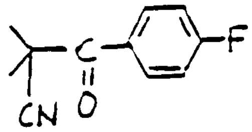

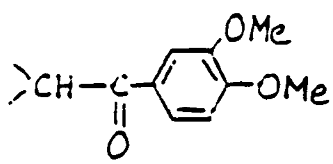

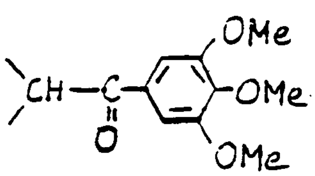

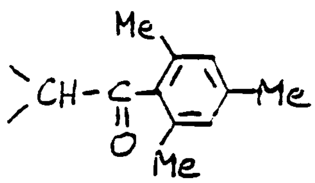

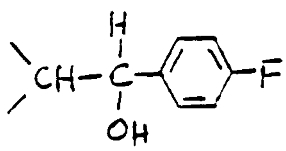

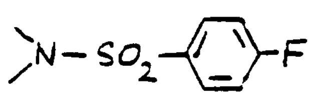

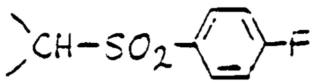

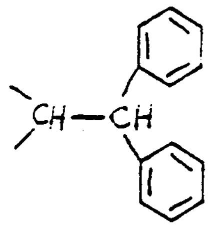

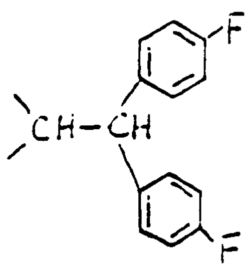

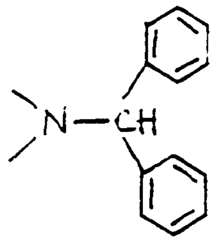

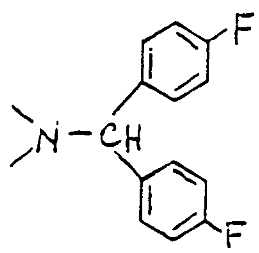

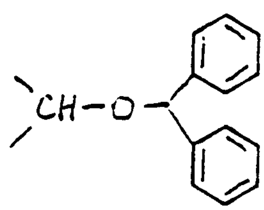

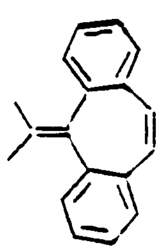

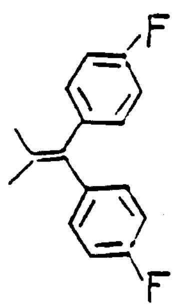

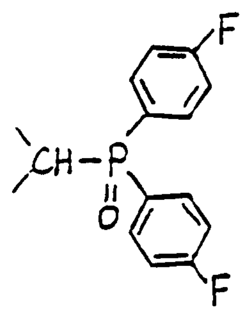

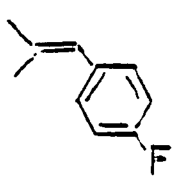

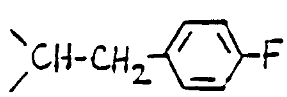

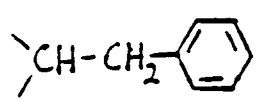,

wherein the group A is represented by any of the following: >CH-, >C=, >N- or >C(CN)-, in which one of the bonds is linked to the group C in formula I or carries hydrogen;
C is hydrogen, methyl, ethyl, propyl, butyl, pentyl or hexyl, or C is bonded to A to form a methylene, or ethylene or trimethylene bridge; Q is selected from the following (II-VII):

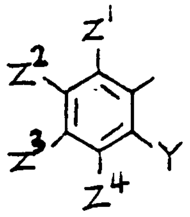

(II)

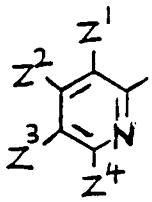

(III)

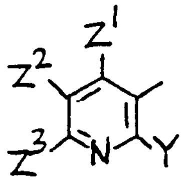

(IV)

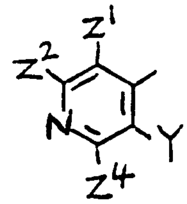

(V)

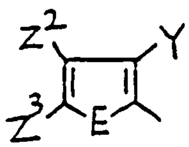

(VI)

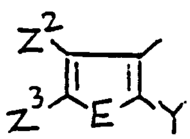

(VII)

wherein E is a nitrogen atom, -NH or substituted -NH, an oxygen atom or a sulfur atom; Y, $Z^1$, $Z^2$, $Z^3$ and $Z^4$ are independently selected from hydrogen and alkyl of from 1 to 6 carbon atoms, alkyl of from 1 to 6 carbon atoms substituted with one or more halogen, oxygen, sulfur, nitrogen or phosphorus atoms, aralkyl of from 6 to 12 carbon atoms, aralkyl of from 6 to 12 carbon atoms substituted with one or more halogen, oxygen, sulfur, nitrogen or phosphorus atoms, aryl of from 6 to 12 carbon atoms and aryl of from 6 to 12 carbon atoms substituted with one or more halogen, oxygen, sulfur, nitrogen or phosphorus atoms; Y is optionally bonded to X to provide a linking group in which the linkage is formed by an alkylene bridge or an alkylene bridge substituted with one or more halogen, oxygen, sulfur, nitrogen or phosphorus atoms and/or at least one oxygen, sulfur or nitrogen atom; and $Z^1$ and $Z^2$, $Z^2$ and $Z^3$, or $Z^3$ and $Z^4$ are optionally bonded to each other to provide a linking group in which the linkage is formed by an alkylene bridge or an alkylene bridge substituted with one or more halogen, oxygen, sulfur, nitrogen or phosphorous atoms and/or at least one oxygen, sulfur or nitrogen atom;
X is an organic group selected from the following (VIII-XI):

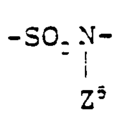

(VIII)

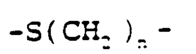

(IX)

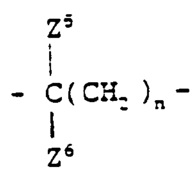

(X)

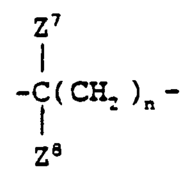

(XI)

wherein, n represents an integer from 0 to 9; $Z^5$ and $Z^6$ are independently hydrogen, alkyl or aralkyl, or alkyl or aralkyl substituted with one or more hetero atoms and/or with one or more substituents optionally containing one or more hetero atoms, $Z^7$ is an alkyl group of from 1 to 20 carbon atoms, or an alkyl group of from 1 to 20 carbon atoms substituted with one or more hetero atoms and/or with one or more substituents optionally containing one or more hetero atoms; and $Z^8$ is hydrogen, cyano, amino, nitro, carboxyl, ester or aminocarbonyl.

2. The use according to Claim 1 wherein $Z^1$, $Z^2$, $Z^3$ and $Z^4$ are independently selected from hydrogen, halogen, hydroxyl, amino, hydroxyamino, nitro, cyano, alkyl, aralkyl, aryl, alkoxy, aralkyloxy, aryloxy, alkylamino, aralkylamino, arylamino, acylamino, acyloxy, aminosulfonyl and any of said alkyl, aralkyl, aryl, alkoxy, aralkyloxy, aryloxy, alkylamino, aralkylamino, arylamino, acylamino acyloxy and aminosulfonyl substituted with one or more halogen, oxygen, sulfur, nitrogen or phosphorus atoms.

**3.** The use according to Claim 1, wherein Q is selected from the following:

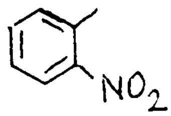 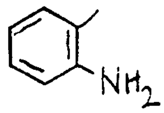 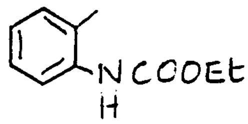 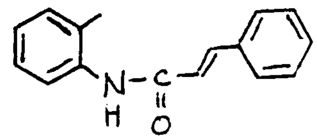

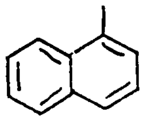 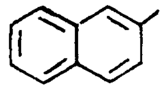 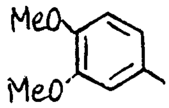 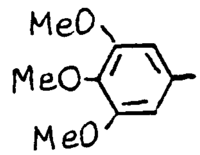

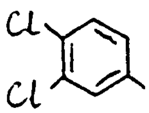 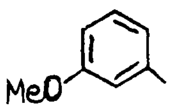 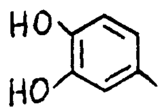 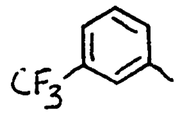

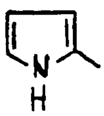 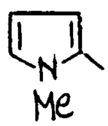 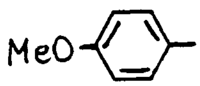 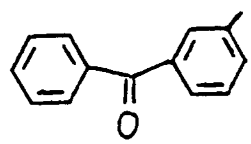

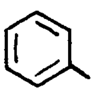 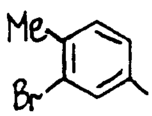 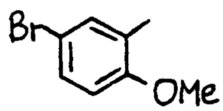 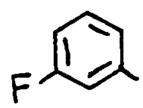

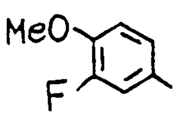 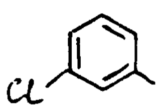 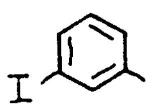 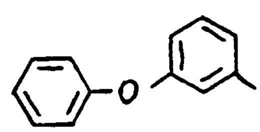

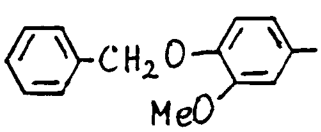 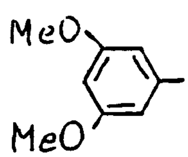 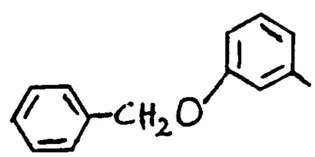

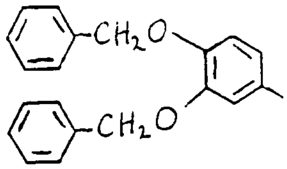
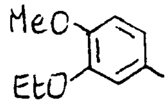
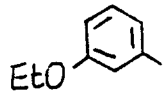

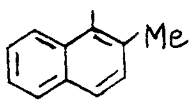
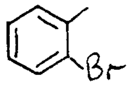
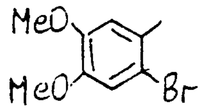
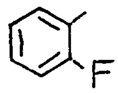

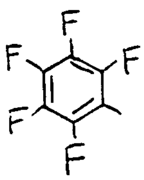
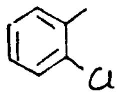
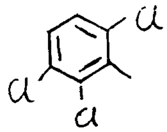
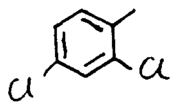
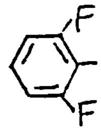

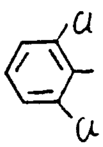
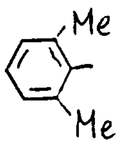
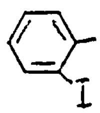
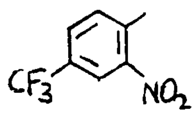
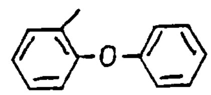

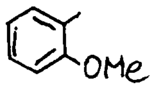
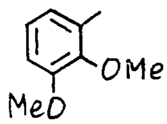
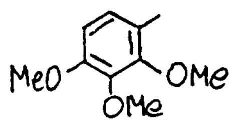
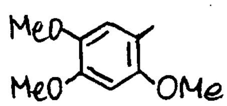

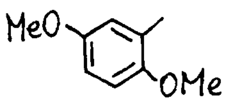
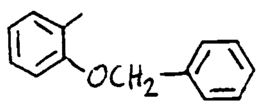
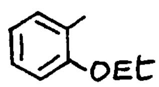
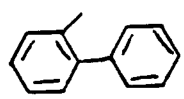

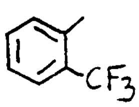
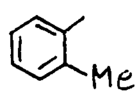
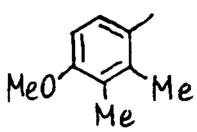
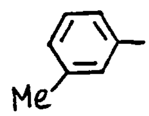
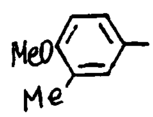

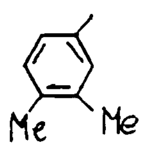
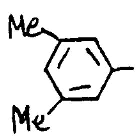
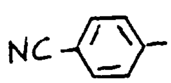
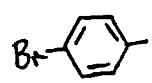

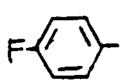
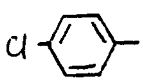
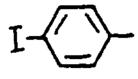
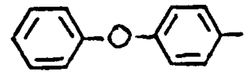

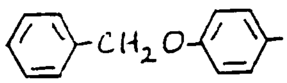
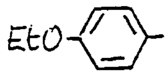
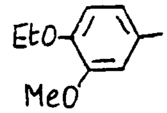
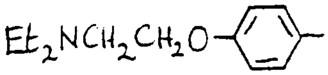
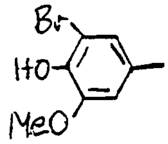
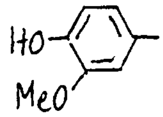
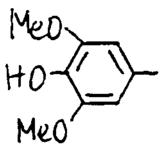
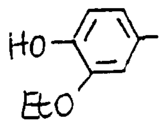
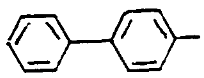
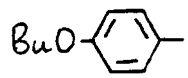
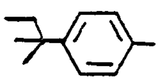
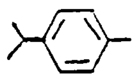
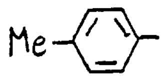
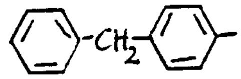
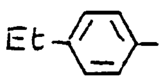
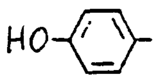
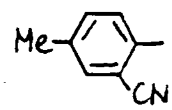
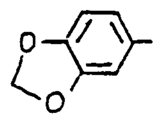
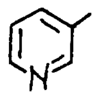
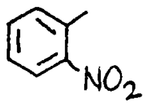
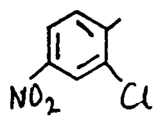
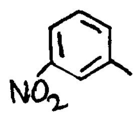
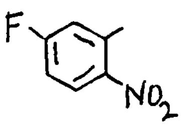
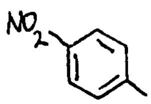
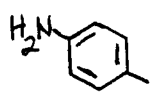
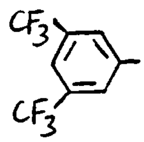
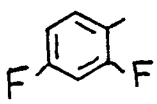
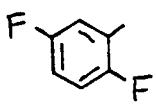
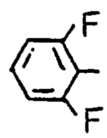
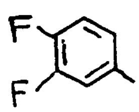
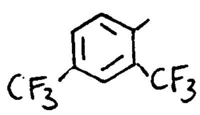
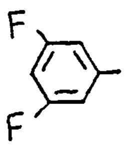
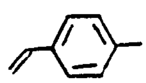
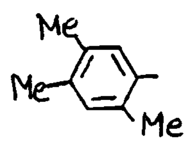

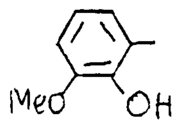
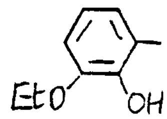
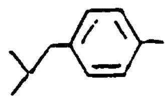

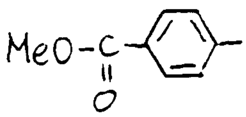
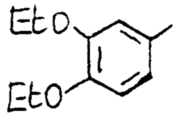
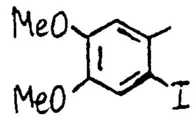

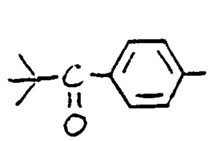
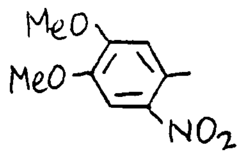
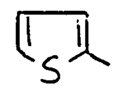

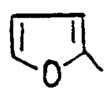
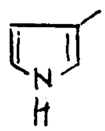
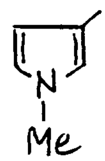
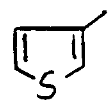

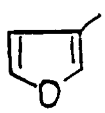

4. The use according to claim 1, wherein the ethylamine derivative is selected from the following:
1-[2-(2-Nitrobenzensulfonyl)aminoethyl]-4-(4-fluorobenzoyl)piperidine
1-[2-(2-Aminobenzensulfonyl)aminoethyl]-4-(4-fluorobenzoyl)piperidine
1-[2-(2-Ethoxycarbonylaminobenzenesulfonyl)aminoethyl]-4-(4-fluorobenzoyl)piperidine
5,6-Benzo-2,4-diaza-2-{2-[4-(4-fluorobenzoyl)-1-piperidinyl]ethyl}tetrahydrothiopyrane-1,1-dioxide
2-(3,4-Dimethoxyphenyl)-5-[4-(4-fluorobenzoyl)-1-piperidinyl]-2-isopropylvaleronitrile
4-(4-Fluorobenzoyl)-1-(4-phenylbutyl)piperidine
1-{3-[2-Cinnamoylaminophenylthio]propyl}-4-(4-fluorobenzoyl)piperidine
1-{3-(2-Aminophenylthio)propyl]-4-(4-fluorobenzoyl)piperidine
2-(3,4-Dimethoxyphenyl)-5-[4-(4-fluorobenzoyl)-1-piperidinyl]valeronitrile
4-(4-Fluorobenzoyl)-1-(5-phenylpentyl)piperidine
5-[4-(4-Fluorobenzoyl)-1-piperidinyl]-2-isopropyl-2-(3,4,5-trimethoxyphenyl)valeronitrile
2-(3,4-Dimethoxyphenyl)-2-dodecyl-5-[4-(4-fluorobenzoyl)-1-piperidinyl]valeronitrile
2-(3,4-Dimethoxyphenyl)-2-{3-[4-(4-fluorobenzoyl)-1-piperidinyl]-1-propyl}-1-3-dithiane-1,1,3,3-tetraoxide
5-[4-(3,4-Dimethoxybenzoyl)-1-piperidinyl]-2-(3,4-dimethoxyphenyl)-2-isopropylvaleronitrile
2-(3,4-Dichlorophenyl)-5-[4-(4-fluorobenzoyl)-1-piperidinyl]-2-isopropylvaleronitrile
2-(3-Benzoylphenyl)-5-[4-(4-fluorobenzoyl)-1-piperidinyl]-2- isopropylvaleronitrile
5,6-Dimethoxy-1-{3-[4-(4-fluorobenzoyl)-1-piperidinyl]propyl}-1-indanenitrile
2-(3,4-Dihydroxyphenyl)-2-dodecyl-5-[4-(4-fluorobenzoyl)-1-piperidinyl]valeronitrile
2-(3,4-Dimethoxyphenyl)-2-dodecyl-5-[4-(α-hydroxy-4-fluorobenzoyl)-1-piperidinyl]valeronitrile
5-[4-(4-Fluorobenzoyl)-1-piperidinyl]-2-(1-methylpyrrole-2-yl)valeronitrile

2-Butyl-2-(3,4-dimethoxyphenyl)-5-[4-(4-fluorobenzoyl)-1-piperidinyl]valeronitrile
2-(3,4-Dimethoxyphenyl)-5-[4-(4-fluorobenzoyl)-1-piperidinyl]-2-octylvaleronitrile
5-[4-(4-Fluorobenzoyl)-1-piperidinyl]-2-isopropyl-2-(1-methylpyrrole-2-yl)valeronitrile
2-(3,4-Dimethoxyphenyl)-5-[4-(4-fluorobenzoyl)-1-piperidinyl]-2-hexadecylvaleronitrile
2-(3,4-Dimethoxyphenyl)-5-[4-(4-fluorobenzoyl)-1-piperidinyl]-2-methylvaleronitrile
2-(3,4-Dimethoxyphenyl)-2-ethyl-5-[4-(4-fluorobenzoyl)-1-piperidinyl]valeronitrile
2-(3,4-Dimethoxyphenyl)-5-[4-(4-fluorobenzoyl)-1-piperidinyl]-2-propylvaleronitrile
2-(3,4-Dimethoxyphenyl)-5-[4-(4-fluorobenzoyl)-1-piperidinyl]-2-pentylvaleronitrile
2-(3,4-Dimethoxyphenyl)-5-[4-(4-fluorobenzoyl)-1-piperidinyl]-2-hexylvaleronitrile
2-(3,4-Dimethoxyphenyl)-5-[4-(4-fluorobenzoyl)-1-piperidinyl]-2-heptylvaleronitrile
2-(3,4-Dimethoxyphenyl)-5-[4-(4-fluorobenzoyl)-1-piperidinyl]-2-nonylvaleronitrile
2-(3,4-Dimethoxyphenyl)-5-[4-(α-hydroxy-4-fluorobenzyl)-1-piperidinyl]-2-isopropylvaleronitrile
1-(4-Phenylbutyl)-α-(4-fluorophenyl)-4-piperidinemethanol L-(+)-tartarate
2,2-Diphenyl)-5-[4-(4-fluorobenzoyl)-1-piperidinyl]valeronitrile
5-{4-[Bis(4-fluorophenyl)methylene]-1-piperidinyl}-2-(3,4-dimethoxyphenyl)-2-isopropylvaleronitrile
2-(3,4-Dimethoxyphenyl)-6-[4-(4-fluorobenzoyl)-1-piperidinyl]-2-isopropylhexanenitrile
2-(3,4-Dimethoxyphenyl)-2-isopropyl-5-[4-(2,4,6-trimethylbenzoyl)-1-piperidinyl]valeronitrile
2-Dodecyl-5-[4-(4-fluorobenzoyl)-1-piperidinyl]-2-(3-methoxyphenyl)valeronitrile
5-[4-(4-Fluorobenzoyl)-1-piperidinyl]-2-phenylvaleronitrile
2-(3-Chloropropyl)-5-[4-(4-fluorobenzoyl)-1-piperidinyl]-2-phenylvaleronitrile
2-(3,4-Dimethoxyphenyl)-7-[4-(4-fluorobenzoyl)-1-piperidinyl]-2-isopropylheptanenitrile
2-(3,4-Dimethoxyphenyl)-5-[4-(4-fluorobenzoyl)-1-piperidinyl]-2-phenylthiovaleronitrile
5-[4-(4-Fluorobenzoyl)-1-piperidinyl]-2-isopropyl-2-(1-naphthyl)valeronitrile
5-[4-(4-Fluorobenzoyl)-1-piperidinyl]-2-isopropyl-2-(2-naphthyl)valeronitrile
2-(3,4-Dimethoxyphenyl)-5-[4-(4-fluorophenyl)methylene-1-piperidinyl]-2-isopropylvaleronitrile
5-[4-(4-Fluorobenzoyl)-1-piperidinyl]-2-isopropyl-2-(3-trifluoromethylphenyl)valeronitrile
2-(3,4-Dimethoxyphenyl)-8-[4-(4-fluorobenzoyl)-1-piperidinyl]-2-isopropyloctanenitrile
2-Decyl-2-(3,4-dimethoxyphenyl)-5-[4-(4-fluorobenzoyl)-1-piperidinyl]valeronitrile
2-(3,4-Dimethoxyphenyl)-5-[4-(4-fluorobenzoyl)-1-piperidinyl]-2-undecylvaleronitrile
2-(3,4-Dimethoxyphenyl)-5-[4-(4-fluorobenzoyl)-1-piperidinyl]-2-tridecylvaleronitrile
2-(3,4-Dimethoxyphenyl)-5-[4-(4-fluorobenzoyl)-1-piperidinyl]-2-tetradecylvaleronitrile
2-(3,4-Dimethoxyphenyl)-5-[4-(4-fluorobenzoyl)-1-piperidinyl]-2-pentadecylvaleronitrile
2-Allyl-2-(3,4-dimethoxyphenyl)-5-[4-(4-fluorobenzoyl)-1-piperidinyl]valeronitrile
2-Cyclopropyl-2-(3,4-dimethoxyphenyl)-5-[4-(4-fluorobenzoyl)-1-piperidinyl]valeronitrile
2-Cyclobutyl-2-(3,4-dimethoxyphenyl)-5-[4-(4-fluorobenzoyl)-1-piperidiyl]valeronitrile
2-Cyclopentyl-2-(3,4-dimethoxyphenyl)-5-[4-(4-fluorobenzoyl)-1-piperidinyl]valeronitrile
2-Cyclohexyl-2-(3,4-dimethoxyphenyl)-5-[4-(4-fluorobenzoyl)-1-piperidinyl]valeronitrile
2-Cyclooctyl-2-(3,4-dimethoxyphenyl)-5-[4-(4-fluorobenzoyl)-1-piperidinyl]valeronitrile
2-Benzyl-2-(3,4-dimethoxyphenyl)-5-[4-(4-fluorobenzoyl)-1-piperidinyl]valeronitrile
2-(3,4-Dimethoxyphenyl)-4-[4-(4-fluorobenzoyl)-1-piperidinyl]-2-dodecylbutyronitrile
2-(3,4-Dimethoxyphenyl)-6-[4-(4-fluorobenzoyl)-1-piperidinyl]-2-dodecylhexanenitrile
2-(3,4-Dimethoxyphenyl)-7-[4-(4-fluorobenzoyl)-1-piperidinyl]-2-dodecylheptanenitrile
2-(3,4-Dimethoxyphenyl)-8-[4-(4-fluorobenzoyl)-1-piperidinyl]-2-dodecyloctanenitrile
5-{4-[Bis(4-fluorophenyl)methylene]-1-piperidinyl}-2-(3,4-dimethoxyphenyl)-2-dodecylvaleronitrile
2-(3,4-Dimethoxyphenyl)-5-[4-(4-fluorobenzyl)-1-piperidinyl]-2-dodecylvaleronitrile
5-(4-Benzyl-1-piperidinyl)-2-(3,4-dimethoxyphenyl)-2-dodecylvaleronitrile
5-(4-Benzyl-1-piperidinyl)-2-(3,4-dimethoxyphenyl)-2-dodecylvaleronitrile
2-(3,4-Dimethoxyphenyl)-5-[4-(4-fluorobenzyl)-1-piperidinyl]-2-dodecylvaleronitrile
5-[4-(3,4-Dimethoxybenzoyl)-1-piperidinyl]-2-(3,4-dimethoxyphenyl)-2-dodecylvaleronitrile
2-(3,4-Dichlorophenyl)-2-dodecyl-5-[4-(4-fluorobenzyl)-1-piperidinyl]valeronitrile
2-(3,4-Dimethoxyphenyl)-2-dodecyl-5-[4-(2,4,6-trimethylbenzoyl)-1-piperidinyl]valeronitrile
5-[4-(4-Fluorobenzoyl)-1-piperidinyl]-2-dodecyl-2-(1-naphthyl)valeronitrile
5-[4-(4-Fluorobenzoyl)-1-piperidinyl]-2-dodecyl-2-(2-naphthyl)valeronitrile
2-(3,4-Dimethoxyphenyl)-2-dodecyl-5-[4-(4-fluorobenzyl)methylene-1-piperidinyl]valeronitrile
5-[4-(4-Fluorobenzoyl)-1-piperidinyl]-2-dodecyl-2-(3-trifluoromethylphenyl)valeronitrile
5-[4-(4-Fluorobenzoyl)-1-piperidinyl]-2-dodecyl-2-(1-methylpyrrole-2-yl)valeronitrile
2-(3,4-Dimethoxyphenyl)-5-[4-(α-hydroxy-4-fluorobenzyl)-1-piperidinyl]-2-methylvaleronitrile
2-(3,4-Dimethoxyphenyl)-2-ethyl-5-[4-(α-hydroxy-4-fluorobenzyl)-1-piperidinyl]valeronitrile

2-(3,4-Dimethoxyphenyl)-5-[4-(α-hydroxy-4-fluorobenzyl)-1-piperidinyl]-2-propylvaleronitrile
2-Butyl-2-(3,4-dimethoxyphenyl)-5-[4-(α-hydroxy-4-fluorobenzyl)-1-piperidinyl]valeronitrile
2-(3,4-Dimethoxyphenyl)-5-[4-(α-hydroxy-4-fluorobenzyl)-1-piperidinyl]-2-pentylvaleronitrile
2-(3,4-Dimethoxyphenyl)-2-hexyl-5-[4-(α-hydroxy-4-fluorobenzyl)-1-piperidinyl]valeronitrile
2-(3,4-Dimethoxyphenyl)-2-heptyl-5-[4-(α-hydroxy-4-fluorobenzyl)-1-piperidinyl]valeronitrile
2-(3,4-Dimethoxyphenyl)-5-[4-(α-hydroxy-4-fluorobenzyl)-1-piperidinyl]-2-octylvaleronitrile
2-(3,4-Dimethoxyphenyl)-5-[4-(α-hydroxy-4-fluorobenzyl)-1-piperidinyl]-2-nonylvaleronitrile
2-Decyl-2-(3,4-dimethoxyphenyl)-5-[4-(α-hydroxy-4-fluorobenzyl)-1-piperidinyl]valeronitrile
2-(3,4-Dimethoxyphenyl)-5-[4-(α-hydroxy-4-fluorobenzyl)-1-piperidinyl]-2-undecylvaleronitrile
2-(3,4-Dimethoxyphenyl)-5-[4-(α-hydroxy-4-fluorobenzyl)-1-piperidinyl]undecylvaleronitrile
2-(3,4-Dimethoxyphenyl)-4-[4-(α-hydroxy-4-fluorobenzyl)-1-piperidinyl]butyronitrile
2-(3,4-Dimethoxyphenyl)-6-[4-(α-hydroxy-4-fluorobenzyl)-1-piperidinyl]hexanenitrile
2-(3,4-Dimethoxyphenyl)-7-[4-(α-hydroxy-4-fluorobenzyl)-1-piperidinyl]heptanenitrile
2-(3,4-Dimethoxyphenyl)-8-[4-(α-hydroxy-4-fluorobenzyl)-1-piperidinyl]octanenitrile
5-{3-[4-(4-Fluorobenzoyl)-1-piperidinyl]propyl}-2,3-dihydro-3-hydroxy-2--phenyl-1,5-benzothiazepine-4-(5H)-one L-(+)-tartarate
5-{3-[4-(4-Fluorobenzoyl)-1-piperidinyl]propyl}-2,3-dihydro-3-hydroxy-2--phenyl-1,5-benzothiazepine-4-(5H)-one acetate L-(+)-tartarate
4-[4-(4-Fluorobenzoyl)-1-piperidinyl]-(3',4'-dimethoxyphenyl)butyrophenone
4-[4-(5H-Dibenzo[a,d]cyclohepten-5-ylidene)-1-piperidinyl]-4'fluorobutyrophenone
2-(3,4-Dimethoxyphenyl)-2-isopropyl-5-(4-phenyl-1-piperidinyl)valeronitrile
2,2-Bis{3-[4-(4-Fluorobenzoyl)-1-piperidinyl]-1-propyl}-(3-methoxyphenyl)acetonitrile
2-{3-[N-(3,4-Dimethoxyphenyl)-N-methylamino]-1-propyl}-2-(3,4-dimethoxyphenyl)-5-[4-(4-fluorobenzoyl)-1-piperidinyl]valeronitrile
3-{2-[4-(4-Fluorobenzoyl)-1-piperidinyl]-1-ethyl}-5-(2-methoxy-1-ethyl)-2,6-dimethyl-4-(3-nitrophenyl)-1,4-dihydropyridine-3,5-dicarboxylate hydrochloride

5. The use according to any one of claims 1 to 4 wherein the ethylamine derivative is used together with a suitable excipient.

6. The use according to any one of claims 1 to 4 wherein the ethylamine derivative is used together with at least one of a diuretic, a calcium antagonist, a β-blocker, an α-blocker and a converting enzyme inhibitor.

## Patentansprüche

1. Verwendung eines Ethylaminderivats der Formel (I) oder eines Salzes davon zur Herstellung eines antihypertensiv wirksamen Arzneimittels

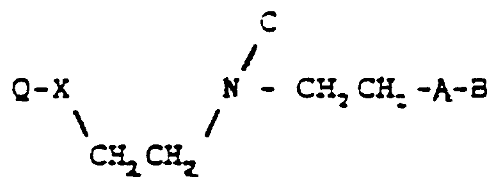

(I)

worin die Struktur der kombinierten Gruppe -A-B unter den folgenden ausgewählt ist:

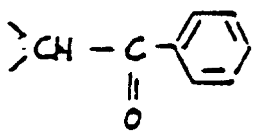
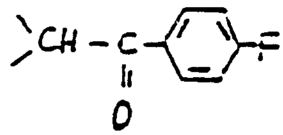
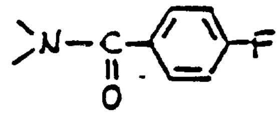

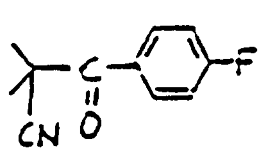
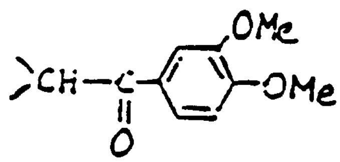

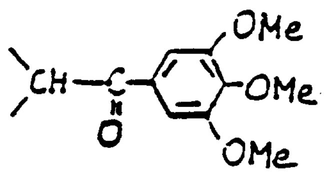
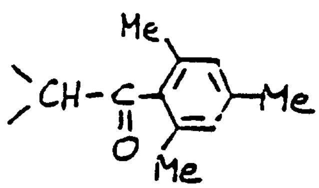

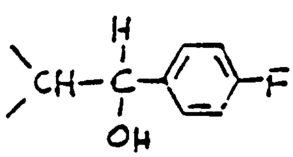
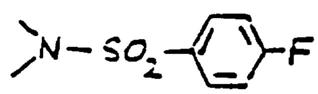

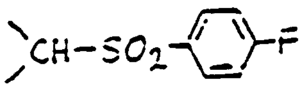
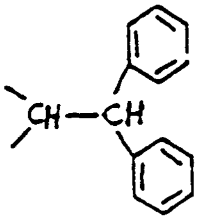

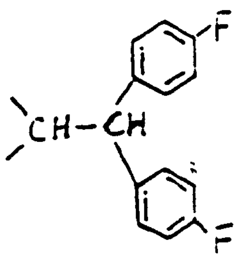
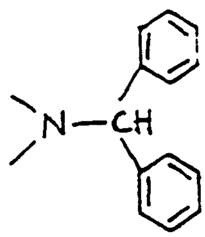

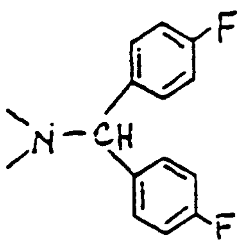
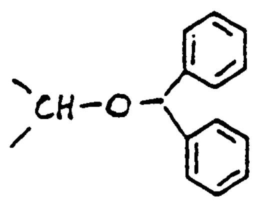

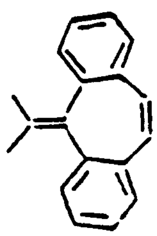
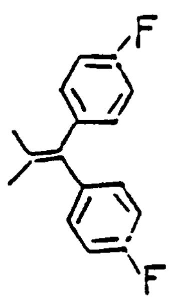
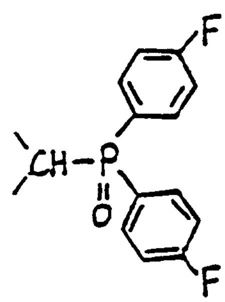

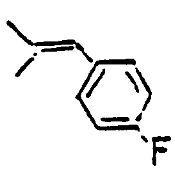
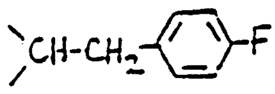
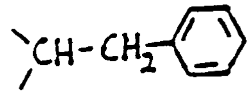 ,

wobei die Gruppe A durch eine der folgenden Strukturen dargestellt ist: >CH-, >C=, >N- oder >C(CN)-, worin eine der Bindungen mit der Gruppe C in Formel I verknüpft ist oder ein Wasserstoffatom trägt,
C Wasserstoff, Methyl, Ethyl, Propyl, Butyl, Pentyl oder Hexyl ist oder C an A unter Bildung einer Methylen-, Ethylen- oder Trimethylen-Brücke gebunden ist, Q unter den folgenden Gruppen (II-VII)

ausgewählt ist:

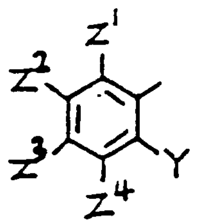
(II)

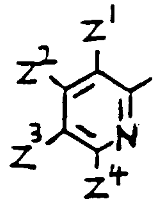
(III)

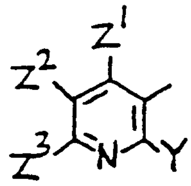
(IV)

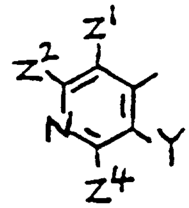
(V)

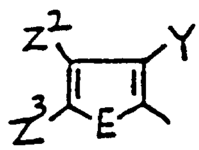
(VI)

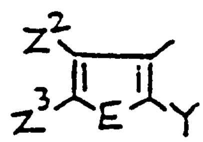
(VII)

worin E ein Stickstoffatom, -NH oder substituiertes -NH, ein Sauerstoffatom oder ein Schwefelatom bedeutet, Y, $Z^1$, $Z^2$, $Z^3$ und $Z^4$ unabhängig voneinander unter Wasserstoff und Alkylgruppen mit 1 bis 6 Kohlenstoffatomen, Alkylgruppen mit 1 bis 6 Kohlenstoffatomen, die mit einem oder mehreren Halogen-, Sauerstoff-, Schwefel-, Stickstoff- oder Phosphoratomen substituiert sind, Aralkylgruppen mit 6 bis 12 Kohlenstoffatomen, Aralkylgruppen mit 6 bis 12 Kohlenstoffatomen, die mit einem oder mehreren Halogen-, Sauerstoff-, Schwefel-, Stickstoff- oder Phosphoratomen substituiert sind, Arylgruppen mit 6 bis 12 Kohlenstoffatomen und Arylgruppen mit 6 bis 12 Kohlenstoffatomen, die mit einem oder mehr Halogen-, Sauerstoff-, Schwefel-, Stickstoff- oder Phosphoratomen substituiert sind, ausgewählt sind, Y gegebenenfalls an X gebunden ist, wobei eine Brückengruppe gebildet wird, in der die Brücke durch eine Alkylenbrücke oder eine Alkylenbrücke, die substituiert ist mit einem oder mehreren Halogen-, Sauerstoff-, Schwefel-, Stickstoff- oder Phosphoratomen und/oder mindestens ein Sauerstoff-, Schwefel- oder Stickstoffatom gebildet wird, und $Z^1$ und $Z^2$, $Z^2$ und $Z^3$ oder $Z^3$ und $Z^4$ gegebenenfalls aneinander gebunden sind, wobei eine Brückengruppe gebildet wird, in der die Brücke durch eine Alkylenbrücke oder eine Alkylenbrücke, die substituiert ist mit einem oder mehr Halogen-, Sauerstoff-, Schwefel-, Stickstoff- oder Phosphoratomen und/oder mindestens ein Sauerstoff-, Schwefel- oder Stickstoffatom gebildet wird, X eine unter den folgenden (VIII) bis (XI) ausgewählte organische Gruppe ist:

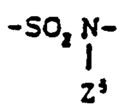
(VIII)

$-S(CH_2)_n-$
(IX)

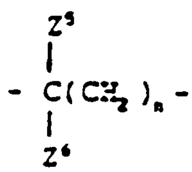
(X)

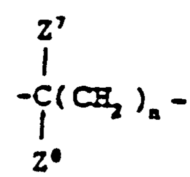
(XI)

worin n eine ganze Zahl von 0 bis 9 bedeutet, $Z^5$ und $Z^6$ unabhängig voneinander für Wasserstoff, Alkyl oder Aralkyl oder Alkyl oder Aralkyl, das mit einem oder mehr Heteroatomen und/oder mit einem oder mehr Substituenten, die gegebenenfalls ein oder mehr Heteroatome enthalten, substituiert ist, stehen, $Z^7$ eine Alkylgruppe mit 1 bis 20 Kohlenstoffatomen oder eine Alkylgruppe mit 1 bis 20 Kohlenstoffatomen ist, die substituiert ist mit einem oder mehr Heteroatomen und/oder mit einem oder mehr Substituenten, die gegebenenfalls ein oder mehr Heteroatome enthalten, und $Z^8$ Wasserstoff, eine Cyan-, Amino-, Nitro-, Carboxyl-, Ester- oder Aminocarbonylgruppe ist.

**2.** Verwendung nach Anspruch 1, wobei $Z^1$, $Z^2$, $Z^3$ und $Z^4$ unabhängig voneinander unter Wasserstoffatomen, Halogenatomen, Hydroxyl-, Amino-, Hydroxyamino-, Nitro-, Cyan-, Alkyl-, Aralkyl-, Aryl-, Alkoxy-, Aralkyloxy-, Aryloxy-, Alkylamino-, Aralkylamino-, Arylamino-, Acylamino-, Acyloxy-, Aminosulfonylgruppen ausgewählt sind und irgendeine dieser Alkyl-, Aralkyl-, Aryl-, Alkoxy-, Aralkyloxy-, Aryloxy-, Alkylamino-, Aralkylamino-, Arylamino-, Acylamino-, Acyloxy- und Aminosulfonylgruppen mit einem oder mehr Halogen-, Sauerstoff-, Schwefel-, Stickstoff- oder Phosphoratomen substituiert ist.

**3.** Verwendung nach Anspruch 1, wobei Q unter den folgenden Gruppen ausgewählt ist:

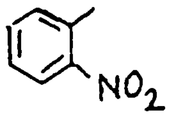
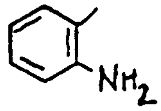
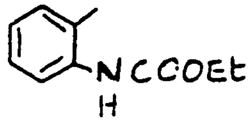
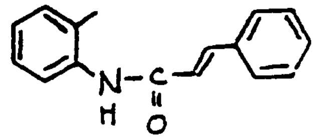

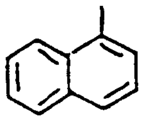
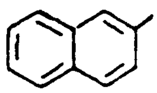
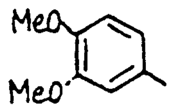
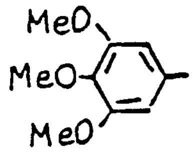

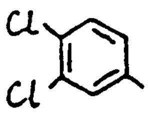
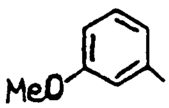
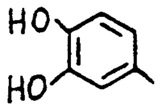
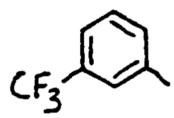

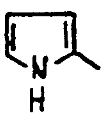
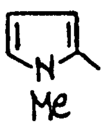
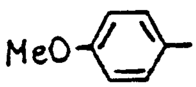
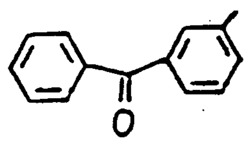

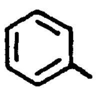
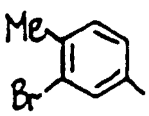
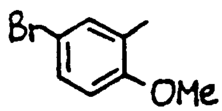
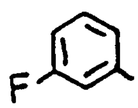

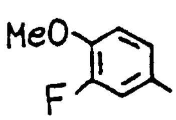
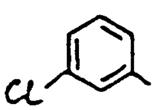
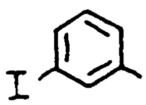
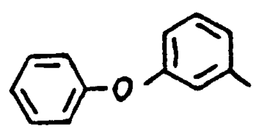

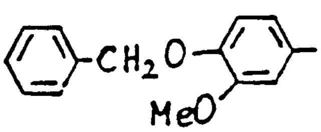
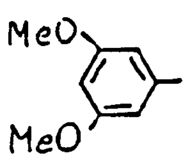
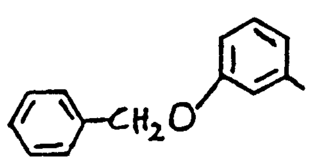

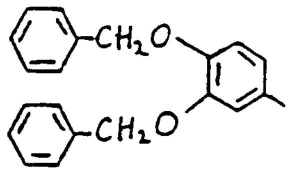 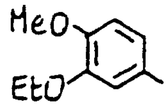 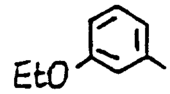

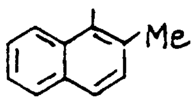 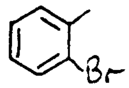 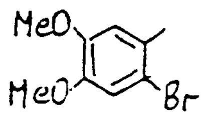 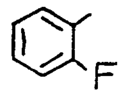

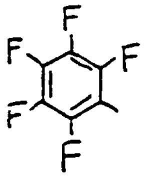 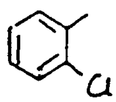 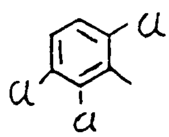 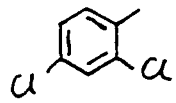 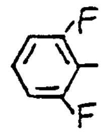

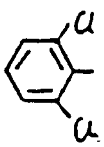 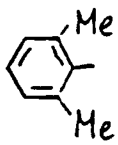 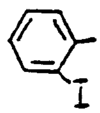 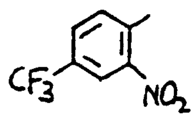 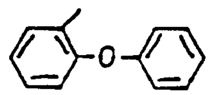

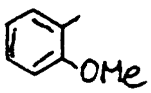 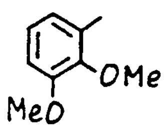 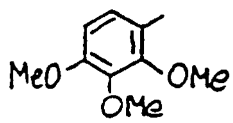 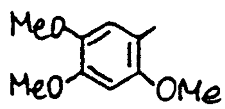

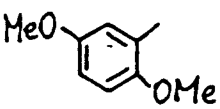 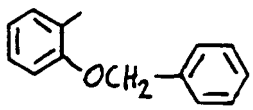 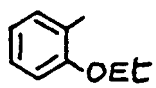 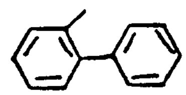

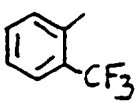 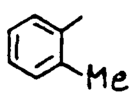 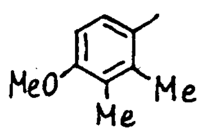 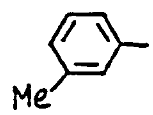 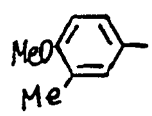

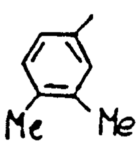 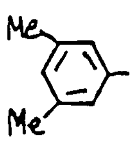 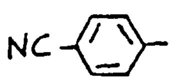 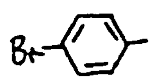

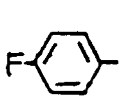 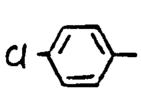 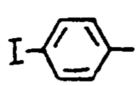 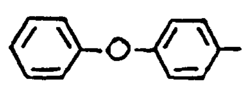

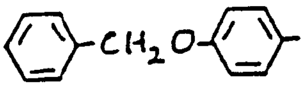
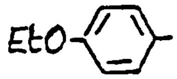
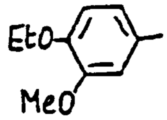

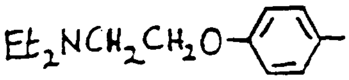
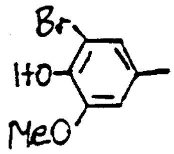
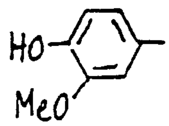

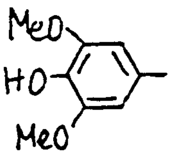
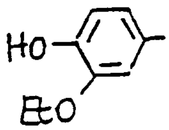
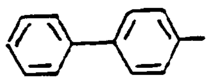
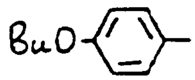

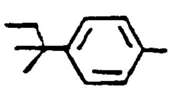
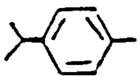
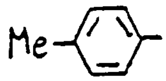
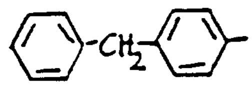

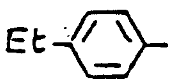
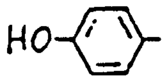
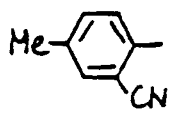
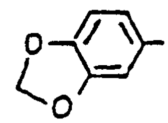

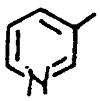
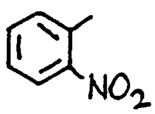
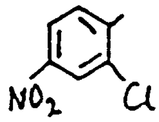
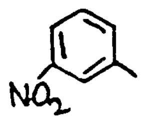

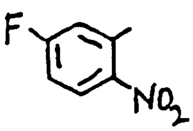
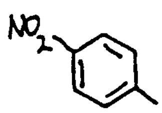
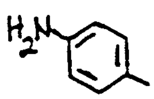
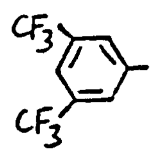

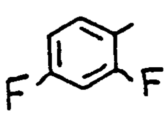
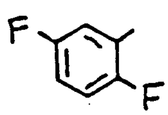
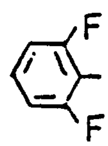
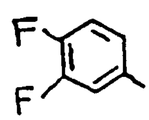

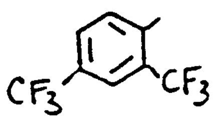
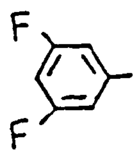
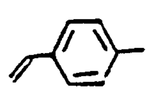
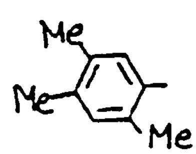

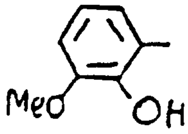 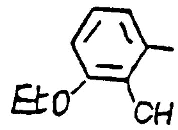 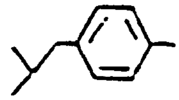

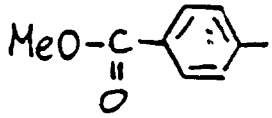 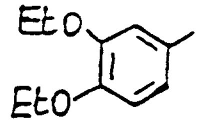 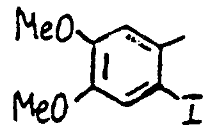

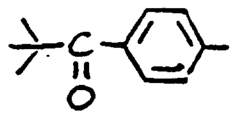 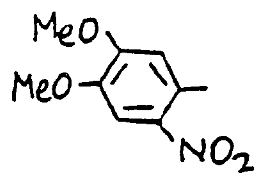 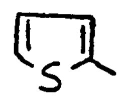

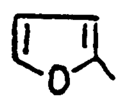 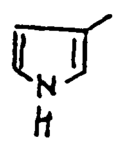 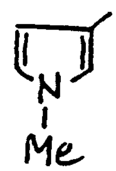 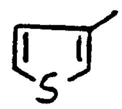

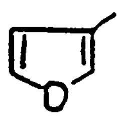

**4.** Verwendung nach Anspruch 1, wobei das Ethylaminderivat unter den folgenden ausgewählt ist:
1-[2-(2-Nitrobenzolsulfonyl)aminoethyl]-4-(4-fluorbenzoyl)piperidin
1-[2-(2-Aminobenzolsulfonyl)aminoethyl]-4-(4-fluorbenzoyl)piperidin
1-[2-(2-Ethoxycarbonylaminobenzolsuifonyi)aminoethyl]-4-(4-fluorbenzoyl)piperidin
5,6-Benzo-2,4-diaza-2-{2-[4-(4-fluorobenzoyl)-1-piperidinyl]ethyl}tetrahydrothiopyran-1,1-dioxid
2-(3,4-dimethoxyphenyl)-5-[4-(4-fluorbenzoyl)-1-piperidinyl]-2-isopropylvaleronitril
4-(4-Fluorbenzoyl)-1-(4-phenylbutyl)piperidin
1-{3-[2-Cinnamoylaminophenylthio]propyl}-4-(4-fluorbenzoyl)Piperidin
1-{3-(2-Aminophenylthio)propyl)-4-(4-fluorbenzoyl)piperidin
2-(3,4-Dimethoxyphenyl)-5-[4-(4-fluorbenzoyl)-1-piperidinyl]valeronitril
4-(4-Fluorbenzoyl)-1-(5-phenylpentyl)piperidin
5-[4-(4-Fluorbenzoyl)-1-piperidinyl]-2-isopropyl-2-(3,4,5-trimethoxyphenyl)valeronitril
2-(3,4-Dimethoxyphenyl)-2-dodecyl-5-[4-(4-fluorbenzoyl)-1-piperidinyl]-valeronitril
2-(3,4-Dimethoxyphenyl)-2-{3-[4-(4-fluorbenzoyl)-1-piperidinyl]-1-propyl}-1,3-dithian-1,1,3,3-tetraoxid
5-[4-(3,4-Dimethoxybenzoyl)-1-piperidinyl]-2-(3,4-dimethoxyphenyl)-2-isopropylvaleronitril
2-(3,4-Dichlorphenyl)-5-[4-(4-fluorbenzoyl)-1-piperidinyl]-2-isopropylvaleronitril
2-(3-Benzoylphenyl)-5-[4-(4-fluorbenzoyl)-1-piperidinyl]-2-isopropylvaleronitril

5,6-Dimethoxy-1-{3-[4-(4-fluorbenzoyl)-1-piperidinyl]propyl}-1-indannitril
2-(3,4-Dihydroxyphenyl)-2-dodecyl-5-[4-(4-fluorbenzoyl)-1-piperidinyl]-valeronitril
2-(3,4-Dimethoxyphenyl)-2-dodecyl-5-[4-(α-hydroxy-4-fluorbenzyl)-1-piperidinyl]valeronitril
5-[4-(4-Fluorbenzoyl)-1-piperidinyl]-2-(1-methylpyrrol-2-yl)valeronitril
2-Butyl-2-(3,4-Dimethoxyphenyl)-5-[4-(4-fluorbenzoyl)-1-piperidinyl]valeronitril
2-(3,4-Dimethoxyphenyl)-5-[4-(4-fluorbenzoyl)-1-piperidinyl]-2-octylvaleronitril
5-[4-(4-Fluorbenzoyl)-1-piperidinyl]-2-isopropyl-2-(1-methylpyrrol-2-yl)valeronitril
2-(3,4-Dimethoxyphenyl)-5-[4-(4-fluorbenzoyl)-1-piperidinyl]-2-hexadecylvaleronitril
2-(3,4-Dimethoxyphenyl)-5-[4-(4-fluorbenzoyl)-1-piperidinyl]-2-methylvaleronitril
2-(3,4-Dimethoxyphenyl)-2-ethyl-5-[4-(4-fluorbenzoyl)-1-piperidinyl]valeronitril
2-(3,4-Dimethoxyphenyl)-5-[4-(4-fluorbenzoyl)-1-piperidinyl]-2-propylvaleronitril
2-(3,4-Dimethoxyphenyl)-5-[4-(4-fluorbenzoyl)-1-piperidinyl]-2-pentylvaleronitril
2-(3,4-Dimethoxyphenyl)-5-[4-(4-fluorbenzoyl)-1-piperidinyl]-2-hexylvaleronitril
2-(3,4-Dimethoxyphenyl)-5-[4-(4-fluorbenzoyl)-1-piperidinyl]-2-heptylvaleronitril
2-(3,4-Dimethoxyphenyl)-5-[4-(4-fluorbenzoyl)-1-piperidinyl]-2-nonylvaleronitril
2-(3,4-Dimethoxyphenyl)-5-[4-(α-hydroxy-4-fluorbenzyl)-1-piperidinyl]-2-isopropylvaleronitril
1-(4-Phenylbutyl)-α-(4-fluorphenyl)-4-piperidinmethanol L-(+)-tartarat
2,2-Diphenyl-5-[4-(4-fluorbenzoyl)-1-piperidinyl]valeronitril
5-{4-[Bis(4-Fluorphenyl)methylen ]-1-piperidinyl}-2-(3,4-dimethoxyphenyl)-2-isopropylvaleronitril
2-(3,4-Dimethoxyphenyl)-6-[4-(4-fluorbenzoyl)-1-piperidinyl]-2-isopropylhexannitril
2-(3,4-Dimethoxyphenyl)-2-isopropyl-5-[4-(2,4,6-trimethylbenzoyl)-1-piperidinyl]valeronitril
2-Dodecyl-5-[4-(4-fluorbenzoyl)-1-piperidinyl]-2-(3-methoxyphenyl)valeronitril
5-[4-(4-Fluorbenzoyl)-1-piperidinyl]-2-phenylvaleronitril
2-(3-Chlorpropyl)-5-[4-(4-fluorbenzoyl)-1-piperidinyl]-2-phenylvaleronitril
2-(3,4-Dimethoxyphenyl)-7-[4-(4-fluorbenzoyl)-1-piperidinyl]-2-isopropylheptannitril
2-(3,4-Dimethoxyphenyl)-5-[4-(4-fluorbenzoyl)-1-piperidinyl]-2-phenylthiovaleronitril
5-[4-(4-Fluorbenzoyl)-1-piperidinyl]-2-isopropyl-2-(1-naphthyl)valeronitril
5-[4-(4-Fluorbenzoyl)-1-piperidinyl]-2-isopropyl-2-(2-naphthyl)valeronitril
2-(3,4-Dimethoxyphenyl)-5-[4-(4-fluorphenyl)methylen-1-piperidinyl]-2-isopropylvaleronitril
5-[4-(4-Fluorbenzoyl)-1-piperidinyl]-2-isopropyl-2-(3-trifluormethylphenyl)valeronitril
2-(3,4-Dimethoxyphenyl)-8-[4-(4-fluorbenzoyl)-1-piperidinyl]-2-isopropyloctannitril
2-Decyl-2-(3,4-dimethoxyphenyl)-5-[4-(4-fluorbenzoyl)-1-piperidinyl]valeronitril
2-(3,4-Dimethoxyphenyl)-5-[4-(4-fluorbenzoyl)-1-piperidinyl]-2-undecylvaleronitril
2-(3,4-Dimethoxyphenyl)-5-[4-(4-fluorbenzoyl)-1-piperidinyl]-2-tridecylvaleronitril
2-(3,4-Dimethoxyphenyl)-5-[4-(4-fluorbenzoyl)-1-piperidinyl]-2-tetradecylvaleronitril
2-(3,4-Dimethoxyphenyl)-5-[4-(4-fluorbenzoyl)-1-piperidinyl]-2-pentadecylvaleronitril
2-Allyl-2-(3,4-dimethoxyphenyl)-5-[4-(4-fluorbenzoyl)-1-piperidinyl]valeronitril
2-Cyclopropyl-2-(3,4-dimethoxyphenyl)-5-[4-(4-fluorbenzoyl)-1-piperidinyl]valeronitril
2-Cyclobutyl-2-(3,4-dimethoxyphenyl)-5-[4-(4-fluorbenzoyl)-1-piperidinyl]valeronitril
2-cyclopentyl-2-(3,4-dimethoxyphenyl)-5-[4-(4-fluorbenzoyl)-1-piperidinyl]valeronitril
2-Cyclohexyl-2-(3,4-dimethoxyphenyl)-5-[4-(4-fluorbenzoyl)-1-piperidinyl]valeronitril
2-Cyclooctyl-2-(3,4-dimethoxyphenyl)-5-[4-(4-fluorbenzoyl)-1-piperidinyl]valeronitril
2-Benzyl-2-(3,4-dimethoxyphenyl)-5-[4-(4-fluorbenzoyl)-1-piperidinyl]valeronitril
2-(3,4-Dimethoxyphenyl)-4-[4-(4-fluorbenzoyl)-1-piperidinyl]-2-dodecylbutyronitril
2-(3,4-Dimethoxyphenyl)-6-[4-(4-fluorbenzoyl)-1-piperidinyl]-2-dodecylhexannitril
2-(3,4-Dimethoxyphenyl)-7-[4-(4-fluorbenzoyl)-1-piperidinyl]-2-dodecylheptannitril
2-(3,4-Dimethoxyphenyl)-8-[4-(4-fluorbenzoyl)-1-piperidinyl]-2-dodecyloctannitril
5-{4-[Bis(4-fluorphenyl)methylen]-1-piperidinyl}-2-(3,4-dimethoxyphenyl)-2-dodecylvaleronitril
2-(3,4-Dimethoxyphenyl)-5-[4-(4-fluorbenzoyl)-1-piperidinyl]-2-dodecylvaleronitril
5-(4-Benzyl-1-piperidinyl)-2-(3,4-dimethoxyphenyl)-2-dodecylvaleronitril
5-(4-Benzoyl-1-piperidinyl)-2-(3,4-dimethoxyphenyl)-2-dodecylvaleronitril
2-(3,4-Dimethoxyphenyl)-5-[4-(4-fluorbenzoyl)-1-piperidinyl]-2-dodecylvaleronitril
5-{4-(3,4-Dimethoxybenzoyl)-1-piperidinyl}-2-(3,4-dimethoxyphenyl)-2-dodecylvaleronitril
2-(3,4-Dichlorphenyl)-2-dodecyl-5-[4-(4-fluorbenzoyl)-1-piperidinyl]valeronitril
2-(3,4-Dimethoxyphenyl)-2-dodecyl-5-[4-(2,4,6-trimethylbenzoyl)-1-piperidinyl]valeronitril
5-[4-(4-Fluorbenzoyl)-1-piperidinyl]-2-dodecyl-2-(1-naphthyl)valeronitril
5-[4-(4-Fluorbenzoyl)-1-piperidinyl]-2-dodecyl-2-(2-naphthyl)valeronitril
2-(3,4-Dimethoxyphenyl)-2-dodecyl-5-[4-(4-fluorphenyl)methylen-1-piperidinyl]valeronitril

5-[4-(4-Fluorbenzoyl)-1-piperidinyl]-2-dodecyl-2-(3-trifluormethylphenyl)valeronitril
5-[4-(4-Fluorbenzoyl)-1-piperidinyl]-2-dodecyl-2-(1-methylpyrrol-2-yl)valeronitril
2-(3,4-Dimethoxyphenyl)-5-[4-(α-hydroxy-4-fluorbenzyl)-1-piperidinyl]-2-methylvaleronitril
2-(3,4-Dimethoxyphenyl)-2-ethyl-5-[4-(α-hydroxy-4-fluorbenzyl)-1-piperidinyl]valeronitril
2-(3,4-Dimethoxyphenyl)-5-[4-(α-hydroxy-4-fluorbenzyl)-1-piperidinyl]-2-propylvaleronitril
2-Butyl-2-(3,4-dimethoxyphenyl)-5-[4-(α-hydroxy-4-fluorbenzyl)-1-piperidinyl]valeronitril
2-(3,4-Dimethoxyphenyl)-5-[4-(α-hydroxy-4-fluorbenzyl)-1-piperidinyl]-2-pentylvaleronitril
2-(3,4-Dimethoxyphenyl)-2-hexyl-5-[4-(α-hydroxy-4-fluorbenzyl)-1-piperidinyl]valeronitril
2-(3,4-Dimethoxyphenyl)-2-heptyl-5-[4-(α-hydroxy-4-fluorbenzyl)-1-piperidinyl]valeronitril
2-(3,4-Dimethoxyphenyl)-5-[4-(α-hydroxy-4-fluorbenzyl)-1-piperidinyl]-2-octylvaleronitril
2-(3,4-Dimethoxyphenyl)-5-[4-(α-hydroxy-4-fluorbenzyl)-1-piperidinyl]-2-nonylvaleronitril
2-Decyl-2-(3,4-dimethoxyphenyl)-5-[4-(α-hydroxy-4-fluorbenzyl)-1-piperidinyl]valeronitril
2-(3,4-Dimethoxyphenyl)-5-[4-(α-hydroxy-4-fluorbenzyl)-1-piperidinyl]-2-undecylvaleronitril
2-(3,4-Dimethoxyphenyl)-5-[4-(α-hydroxy-4-fluorbenzyl)-1-piperidinyl]-undecylvaleronitril
2-(3,4-Dimethoxyphenyl)-4-[4-(α-hydroxy-4-fluorbenzyl)-1-piperidinyl]-butyronitril
2-(3,4-Dimethoxyphenyl)-6-[4-(α-hydroxy-4-fluorbenzyl)-1-piperidinyl]-hexannitril
2-(3,4-Dimethoxyphenyl)-7-[4-(α-hydroxy-4-fluorbenzyl)-1-piperidinyl]-heptannitril
2-(3,4-Dimethoxyphenyl)-8-[4-(α-hydroxy-4-fluorbenzyl)-1-piperidinyl]-octannitril
5-{3-[4-(4-Fluorbenzoyl)-1-piperidinyl]propyl}-2,3-dihydro-3-hydroxy-2-phenyl-1,5-benzothiazepin-4(5H)-on-L-(+)-tartrat
5-{3-[4-(4-Fluorbenzoyl)-1-piperidinyl]propyl}-2,3-dihydro-3-hydroxy-2-phenyl-1,5-benzothiazepin-4(5H)-on-acetat-L-(+)-tartrat
4-[4-(4-Fluorbenzoyl)-1-piperidinyl]-(3',4'-dimethoxyphenyl)butyrophenon
4-[4-(5H-Dibenzo[a,d]cyclohepten-5-yliden)-1-piperidinyl]-4'fluorbutyrophenon
2-(3,4-Dimethoxyphenyl)-2-isopropyl-5-(4-phenyl-1-piperidinyl)valeronitril
2,2-Bis{3-[4-(4-fluorbenzoyl)-1-piperidinyl]-1-propyl}-(3-methoxyphenyl)acetonitril
2-{3-[N-(3,4-Dimethoxyphenyl)-N-methylamino]-1-propyl}-2-(3,4-dimethoxyphenyl)-5-[4-(4-fluorbenzoyl)-1-piperidinyl]valeronitril
3-{2-[4-(4-Fluorbenzoyl)-1-piperidinyl]-1-ethyl]-5-(2-methoxy-1-ethyl)-2,6-dimethyl-4-(3-nitrophenyl)-1,4-dihydropyridin-3,5-dicarboxylat-hydrochlorid

**5.** Verwendung nach einem der Ansprüche 1 bis 4, wobei das Ethylaminderivat zusammen mit einem geeigneten Trägermaterial verwendet wird.

**6.** Verwendung nach einem der Ansprüche 1 bis 4, wobei das Ethylaminderivat zusammen mit mindestens einem der folgenden verwendet wird:einem Diureticum, einem Calcium-Antagonisten, einem β-Blocker, einem α-Blocker und einem Konversions-Enzym-Inhibitor (converting enzyme inhibitor) verwendet wird.

## Revendications

**1.** Utilisation dans la préparation d'un médicament anti-hypertensif d'un dérivé de l'éthylamine de formule (I) ou de l'un de ses sels:

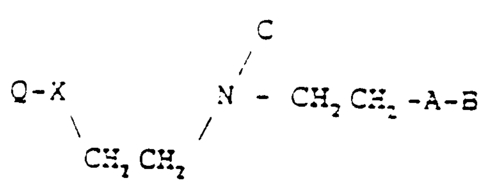

(I)

dans laquelle la structure du groupe -A-B combiné est choisie parmi les suivantes:

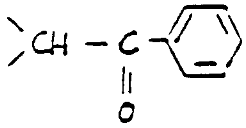

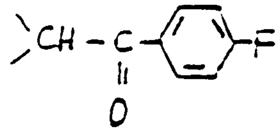

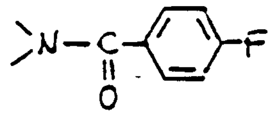

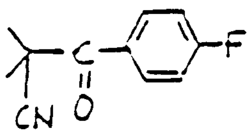

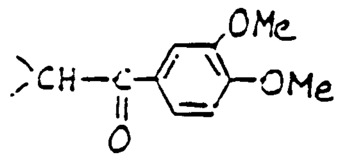

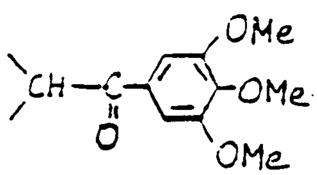

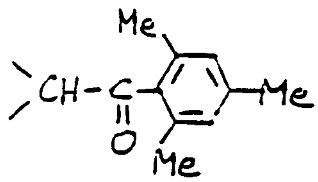

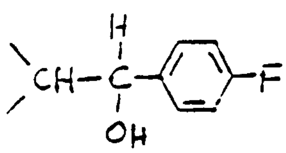

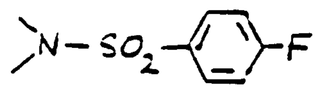

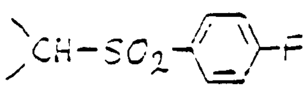
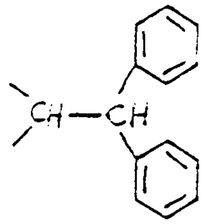

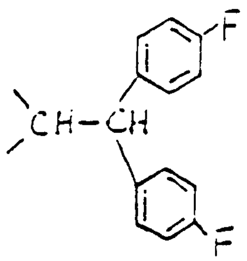
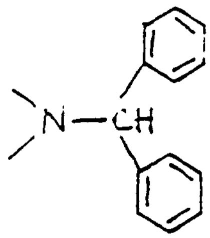

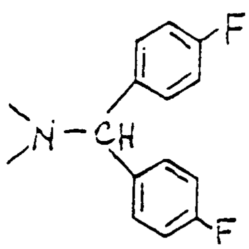
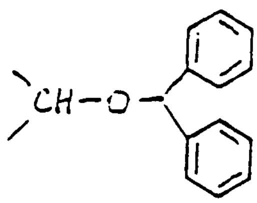

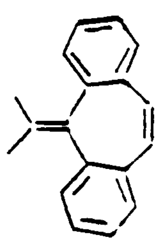
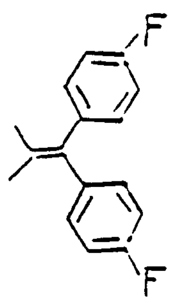
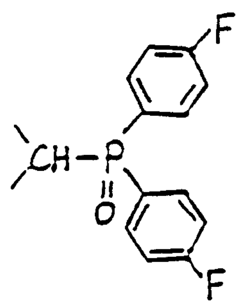

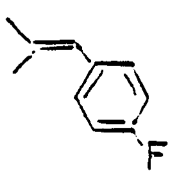
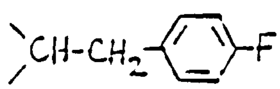
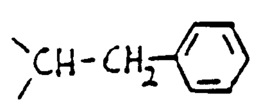,

dans lesquelles le groupe A est représenté par l'un quelconque des suivants : >CH-, >C=, >N- ou >C(CN)-, dans lesquels l'une des liaisons est liée au groupe C de la formule I ou porte un atome d'hydrogène;
C est un atome d'hydrogène ou un groupe méthyle, éthyle, propyle, butyle, pentyle ou hexyle, ou bien C est lié à A pour former un pont méthylène, éthylène ou triméthylène; Q est choisi parmi les groupes

(II) à (VII) suivants :

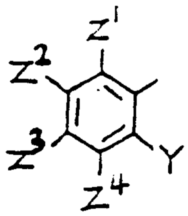
(II)

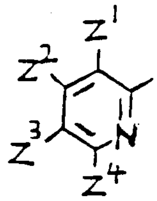
(III)

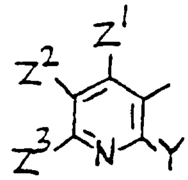
(IV)

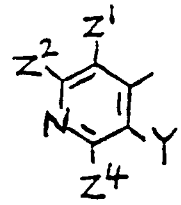
(V)

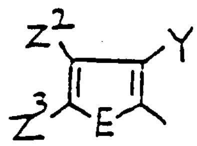
(VI)

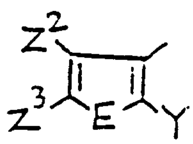
(VII)

dans lesquels E est un atome d'azote, -NH ou -NH substitué, un atome d'oxygène ou un atome de soufre ; Y, $Z^1$, $Z^2$, $Z^3$ et $Z^4$ sont choisis indépendamment parmi l'hydrogène et un groupe alkyle de 1 à 6 atomes de carbone, un groupe alkyle de 1 à 6 atomes de carbone substitué par un ou plusieurs atomes d'halogène, d'oxygène, de soufre, d'azote ou de phosphore, un groupe aralkyle de 6 à 12 atomes de carbone, un groupe aralkyle de 6 à 12 atomes de carbone substitué par un ou plusieurs atomes d'halogène, d'oxygène, de soufre, d'azote ou de phosphore, un groupe aryle de 6 à 12 atomes de carbone et un groupe aryle de 6 à 12 atomes de carbone substitué par un ou plusieurs atomes d'halogène, d'oxygène, de soufre, d'azote ou de phosphore ; Y est éventuellement lié à X pour former un groupe de liaison dans lequel la liaison est formée par un pont alkylène ou un pont alkylène substitué par un ou plusieurs atomes d'halogène, d'oxygène, de soufre, d'azote ou de phosphore et/ou au moins un atome d'oxygène, de soufre ou d'azote ; et $Z^1$ et $Z^2$, $Z^2$ et $Z^3$ ou $Z^3$ et $Z^4$ sont éventuellement liés l'un à l'autre pour former un groupe de liaison dans lequel la liaison est formée par un pont alkylène ou un pont alkylène substitué par un ou plusieurs atomes d'halogène, d'oxygène, de soufre, d'azote ou de phosphore et/ou au moins un atome d'oxygène, de soufre ou d'azote; X est un groupe organique choisi parmi les groupes (VIII) à (XI) suivants :

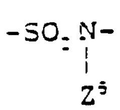
(VIII)

$-S(CH_2)_n-$
(IX)

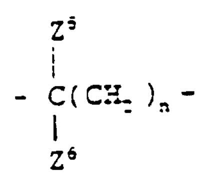
(X)

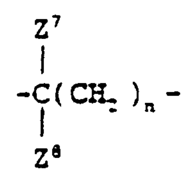
(XI)

dans lesquels n représente un nombre entier de 0 à 9, $Z^5$ et $Z^6$ représentent indépendamment un atome d'hydrogène, un groupe alkyle ou aralkyle, ou un groupe alkyle ou aralkyle substitué par un ou plusieurs hétéroatomes et/ou par un ou plusieurs substituants contenant éventuellement un ou plusieurs hétéroatomes, $Z^7$ est un groupe alkyle de 1 à 20 atomes de carbone ou un groupe alkyle de 1 à 20 atomes de carbone substitué par un ou plusieurs hétéroatomes et/ou par un ou plusieurs substituants contenant éventuellement un ou plusieurs hétéroatomes, et $Z^8$ est un atome d'hydrogène ou un groupe cyano, amino, nitro, carboxyle, ester ou aminocarbonyle.

**2.** Utilisation selon la revendication 1, dans laquelle $Z^1$, $Z^2$, $Z^3$ et $Z^4$ sont choisis indépendamment parmi l'hydrogène, un halogène, un groupe hydroxyle, amino, hydroxyamino, nitro, cyano, alkyle, aralkyle, aryle, alcoxy, aralkyloxy, aryloxy, alkylamino, aralkylamino, arylamino, acylamino, acyloxy, aminosulfonyle et l'un quelconque desdits groupes alkyle, aralkyle, aryle, alcoxy, aralkyloxy, aryloxy, alkylamino, aralkylamino, arylamino, acylamino, acyloxy et aminosulfonyle substitués par un ou plusieurs atomes d'halogène, d'oxygène, de soufre, d'azote ou de phosphore.

**3.** Utilisation selon la revendication 1, dans laquelle Q est choisi parmi les groupes suivants :

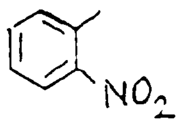
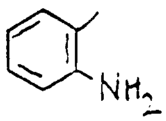
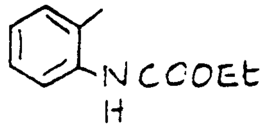
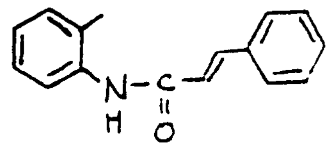

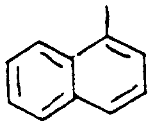 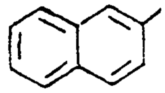 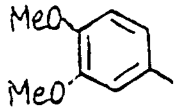 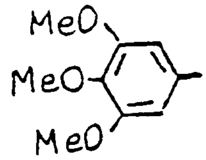

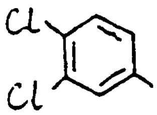 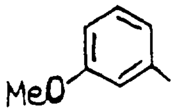 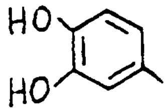 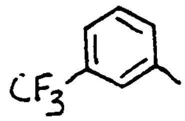

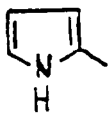 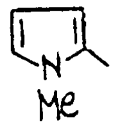 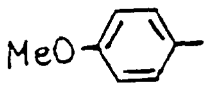 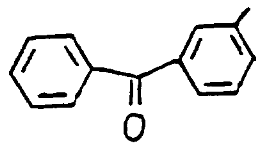

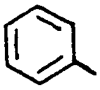 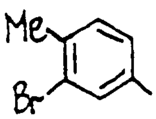 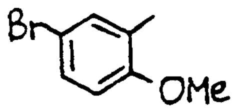 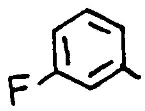

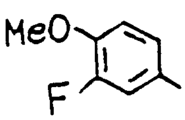 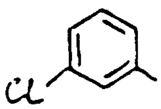 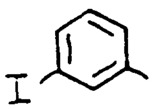 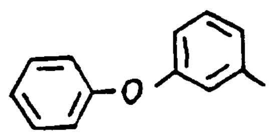

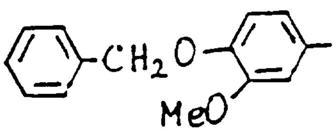 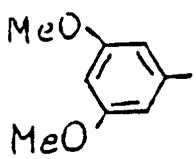 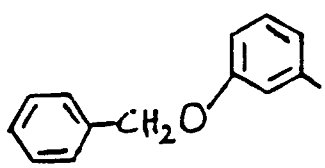

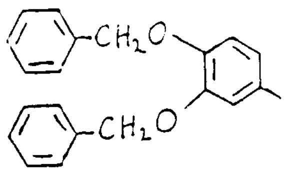 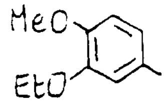 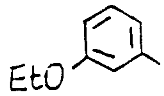

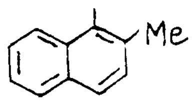 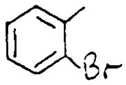 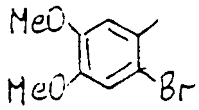 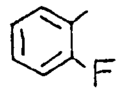

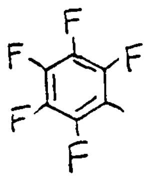 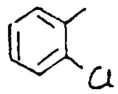 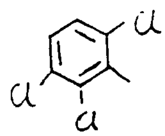 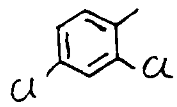 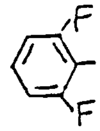

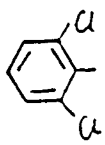 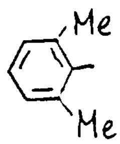 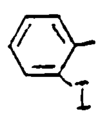 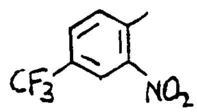 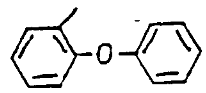

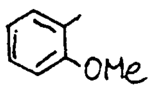 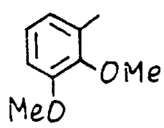 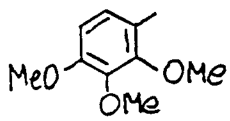 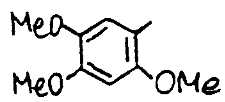

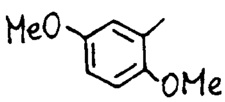 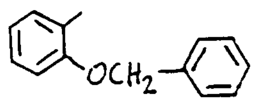 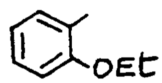 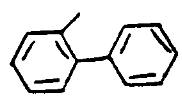

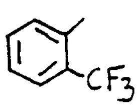 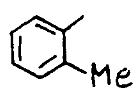 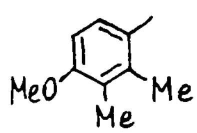 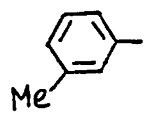 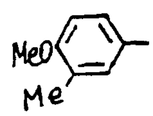

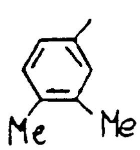 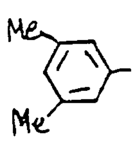 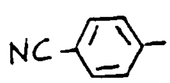 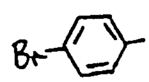

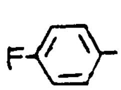 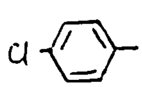 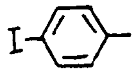 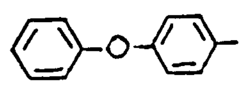

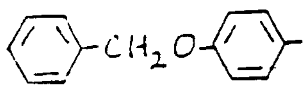 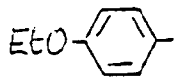 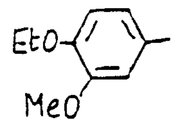

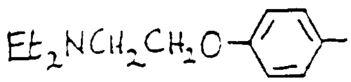 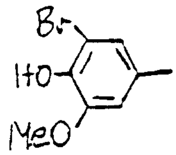 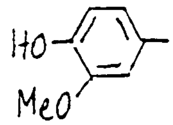

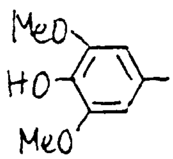 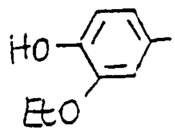 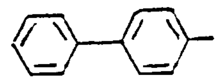 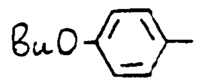

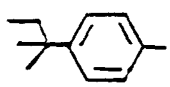 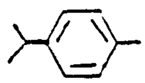 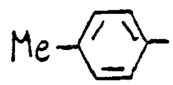 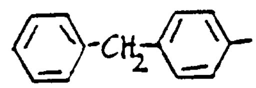

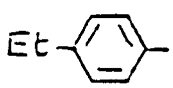 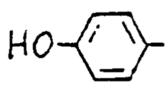 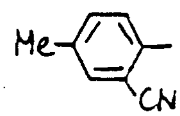 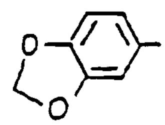

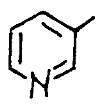 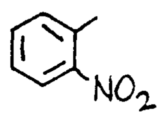 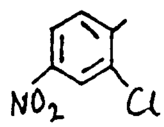 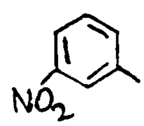

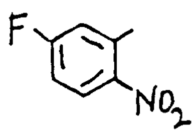 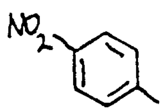 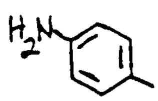 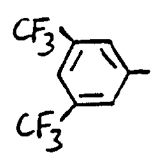

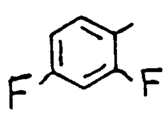 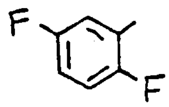 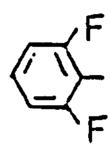 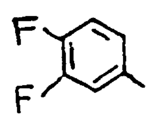

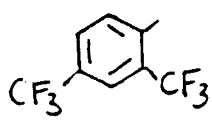 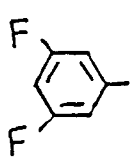 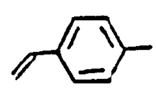 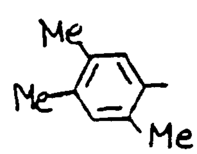

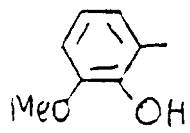
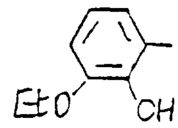
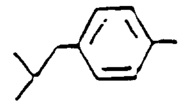

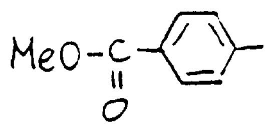
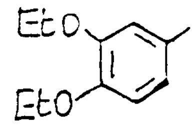
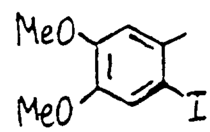

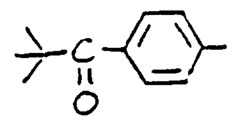
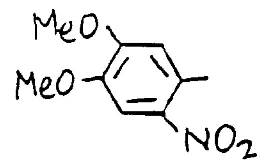
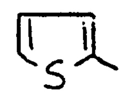

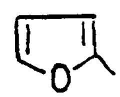
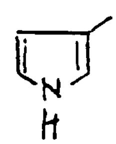
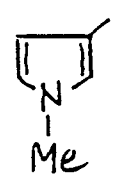
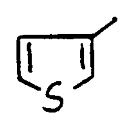

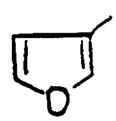

**4.** Utilisation selon la revendication 1, dans laquelle le dérivé de l'éthylamine est choisi parmi les suivants :
1-[2-(2-nitrobenzènesulfonyl)aminoéthyl]-4-(4-fluorobenzoyl)pipéridine,
1-[2-(2-aminobenzènesulfonyl)aminoéthyl]-4-(4-fluorobenzoyl)pipéridine,
1-[2-(2-éthoxycarbonylaminobenzènesulfonyl)aminoéthyl]-4-(4-fluorobenzoyl)pipéridine,
5,6-benzo-2,4-diaza-2-{2-[4-(4-fluorobenzoyl)-1-pipéridinyl]éthyl}tétrahydrothiopyranne-1,1-dioxyde,
2-(3,4-diméthoxyphényl)-5-[4-(4-fluorobenzoyl)-1-pipéridinyl]-2-isopropylvaléronitrile,
4-(4-fluorobenzoyl)-1-(4-phenylbutyl)pipéridine,
1-{3-[2-cinnamoylaminophénylthio]propyl}-4-(4-fluorobenzoyl)pipéridine,
1-{3-[2-aminophénylthio]propyl}-4-(4-fluorobenzoyl)pipéridine,
2-(3,4-diméthoxyphényl)-5-[4-(4-fluorobenzoyl)-1-pipéridinyl]valéronitrile,
4-(4-fluorobenzoyl)-1-(5-phénylpentyl)pipéridine,
5-[4-(4-fluorobenzoyl)-1-pipéridinyl]-2-isopropyl-2-(3,4,5-triméthoxyphényl)valéronitrile,
2-(3,4-diméthoxyphényl)-2-dodécyl-5-[4-(4-fluorobenzoyl)-1-pipéridinyl]valéronitrile,
2-(3,4-diméthoxyphényl)-2-{3-[4-(4-fluorobenzoyl)-1-pipéridinyl]-1-propyl}-1,3-dithiane-1,1,3,3-tétraoxyde,
5-[4-(3,4-diméthoxybenzoyl)-1-pipéridinyl]-2-(3,4-diméthoxyphényl)-2-isopropylvaleronitrile,

2-(3,4-dichlorophényl)-5-[4-(4-fluorobenzoyl)-1-pipéridinyl]-2-isopropylvaléronitrile,
2-(3-benzoylphényl)-5-[4-(4-fluorobenzoyl)-1-piperidinyl]-2-isopropylvaléronitrile,
5,6-diméthoxy-1-{3-[4-(4-fluorobenzoyl)-1-pipéridinyl]propyl}-1-indanenitrile,
2-(3,4-dihydroxyphényl)-2-dodécyl-5-[4-(4-fluorobenzoyl)-1-pipéridinyl]valéronitrile,
2-(3,4-dimethoxyphényl-2-dodécyl-5-[4-(α-hydroxy-4-fluorobenzyl)-1-pipéridinyl]valéronitrile,
5-[4-(4-fluorobenzoyl)-1-pipéridinyl]-2-(1-méthylpyrrol-2-yl)valéronitrile,
2-butyl-2-(3,4-diméthoxyphényl)-5-[4-(4-fluorobenzoyl)-1-pipéridinyl]valéronitrile,
2-(3,4-diméthoxyphényl)-5-[4-(4-fluorobenzoyl)-1-pipèridinyl]-2-oetylvaléronitrile,
5-[4-(4-fluorobenzoyl)-1-pipéridinyl]-2-isopropyl-2-(1-méthylpyrrol-2-yl)valéronitrile,
2-(3,4-dimèthoxyphényl)-5-[4-(4-fluorobenzoyl)-1-pipéridinyl]-2-hexadécylvaléronitrile,
2-(3,4-diméthoxyphényl)-5-[4-(4-fluorobenzoyl)-1-pipéridinyl]-2-méthylvaléronitrile,
2-(3,4-diméthoxyphényl)-2-éthyl-5-[4-(4-fluorobenzoyl)-1-pipéridinyl]valéronitrile,
2-(3,4-diméthoxyphényl)-5-[4-(4-fluorobenzoyl)-1-pipéridinyl]-2-propylvaléronitrile,
2-(3,4-diméthoxyphényl)-5-[4-(4-fluorobenzoyl)-1-pipéridinyl]-2-pentylvaléronitrile,
2-(3,4-diméthoxyphényl)-5-[4-(4-fluorobenzoyl)-1-pipéridinyl]-2-hexylvaléronitrile,
2-(3,4-diméthoxyphényl)-5-[4-(4-fluorobenzoyl)-1-pipéridinyl]-2-heptylvaléronitrile,
2-(3,4-diméthoxyphényl)-5-[4-(4-iluorobenzoyl)-1-pipéridinyl]-2-nonylvaléronitrile,
2-(3,4-diméthoxyphényl)-5-[4-(α-hydroxy-4-fluorobenzoyl)-1-pipéridinyl]-2-isopropylvaléronitrile,
L-( + )-tartrate de 1-(4-phénylbutyl)-α-(4-fluorophényl)-4-pipéridineméthanol,
2,2-diphényl-5-[4-(4-fluorobenzoyl)-1-pipéridinyl]valéronitrile,
5-{4-[bis(4-fluorophényl)méthylène]-1-pipéridinyl}-2-(3,4-diméthoxyphényl)-2-isopropylvaléronitrile,
2-(3,4-diméthoxyphenyl)-6-[4-(4-fluorobenzoyl)-1-pipéridinyl]-2-isopropylhexanenitrile,
2-(3,4-diméthoxyphényl)-2-isopropyl-5-[4-(2,4,6-triméthylbenzoyl)-1-pipéridinyl]valéronitrile,
2-dodécyl-5-[4-(4-fluorobenzoyl)-1-pipéridinyl]-2-(3-méthoxyphényl)valéronitrile,
5-[4-(4-fluorobenzoyl)-1-pipéridinyl]-2-phénylvaléronitrile,
2-(3-chloropropyl)-5-[4-(4-fluorobenzoyl)-1-pipéridinyl]-2-phénylvaléronitrile,
2-(3,4-diméthoxyphényl)-7-[4-(4-fluorobenzoyl)-1-pipéridinyl]-2-isopropylheptanenitrile,
2-(3,4-diméthoxyphényl)-5-[4-(4-fluorobenzoyl)-1-pipéridinyl]-2-phénylthiovaléronitrile,
5-[4-(4-fluorobenzoyl)-1-piperidinyl]-2-isopropyl-2-(1-naphtyl)valéronitrile,
5-[4-(4-fluorobenzoyl)-1-pipéridinyl]-2-isopropyl-2-(2-naphtyl)valèronitrile,
2-(3,4-diméthoxyphényl)-5-[4-(4-fluorophényl)méthylène-1-pipéridinyl]-2-isopropylvaléronitrile,
5-[4-(4-fluorobenzoyl)-1-pipéridinyl]-2-isopropyl-2-(3-trifluorométhylphényl)valéronitrile,
2-(3,4-diméthoxyphényl)-8-[4-(4-fluorobenzoyl)-1-pipéridinyl]-2-isopropyloctanenitrile,
2-décyl-2-(3,4-diméthoxyphényl)-5-[4-(4-fluorobenzoyl)-1-pipéridinyl]valéronitrile,
2-(3,4-diméthoxyphényl)-5-[4-(4-fluorobenzoyl)-1-pipéridinyl]-2-undécylvaléronitrile,
2-(3,4-diméthoxyphényl)-5-[4-(4-fluorobenzoyl)-1-pipéridinyl]-2-tridécylvaléronitrile,
2-(3,4-diméthoxyphényl)-5-[4-(4-fluorobenzoyl)-1-pipéridinyl]-2-tétradécylvaléronitrile,
2-(3,4-diméthoxyphényl)-5-[4-(4-fluorobenzoyl)-1-pipéridinyl]-2-pentadécylvaléronitrile,
2-allyl-2-(3,4-dimethoxyphényl)-5-[4-(4-fluorobenzoyl)-1-pipéridinyl]valéronitrile,
2-cyclopropyl-2-(3,4-diméthoxyphényl)-5-[4-(4-fluorobenzoyl)-1-pipéridinyl]valéronitrile,
2-cyclobutyl-2-(3,4-diméthoxyphényl)-5-[4-(4-fluorobenzoyl)-1-pipéridinyl]valéronitrile,
2-cyclopentyl-2-(3,4-diméthoxyphényl)-5-[4-(4-fluorobenzoyl)-1-pipéridinyl]valéronitrile,
2-cyclohexyl-2-(3,4-diméthoxyphényl)-5-[4-(4-fluorobenzoyl)-1-pipéridinyl]valéronitrile,
2-cyclooctyl-2-(3,4-diméthoxyphényl)-5-[4-(4-fluorobenzoyl)-1-pipéridinyl]valéronitrile,
2-benzyl-2-(3,4-diméthoxyphényl)-5-[4-(4-fluorobenzoyl)-1-pipéridinyl]valéronitrile,
2-(3,4-diméthoxyphényl)-4-[4-(4-fluorobenzoyl)-1-pipéridinyl]-2-dodécylbutyronitrile,
2-(3,4-diméthoxyphényl)-6-[4-(4-fluorobenzoyl)-1-pipéridinyl]-2-dodécylhexanenitrile,
2-(3,4-diméthoxyphényl)-7-[4-(4-fluorobenzoyl)-1-pipéridinyl]-2-dodécylheptanenitrile,
2-(3,4-diméthoxyphényl)-8-[4-(4-fluorobenzoyl)-1-pipéridinyl]-2-dodécyloctanenitrile,
5-{4-[bis(4-fluorophenyl)methylene]-1-piperidinyl}-2-(3,4-dimethoxyphényl)-2-dodécylvaléronitrile,
2-(3,4-diméthoxyphényl)-5-[4-(4-fluorobenzyl)-1-pipéridinyl]-2-dodécylvaléronitrile,
5-(4-benzyl-1-pipéridinyl)-2-(3,4-diméthoxyphényl)-2-dodécylvaléronitrile,
5-(4-benzoyl-1-pipéridinyl)-2-(3,4-dimethoxyphényl)-2-dodecylvaléronitrile,
2-(3,4-diméthoxyphényl)-5-[4-(4-fluorobenzoyl)-1-pipéridinyl]-2dodécylvaléronitrile,
5-[4-(3,4-diméthoxybenzoyl)-1-piperidinyl]-2-(3,4-diméthoxyphényl)-2-dodécylvaléronitrile,
2-(3,4-dichlorophényl)-2-dodécyl-5-[4-(4-fluorobenzoyl)-1-pipéridinyl)valéronitrile,
2-(3,4-diméthoxyphényl)-2-dodécyl-5-[4-(2,4,6-triméthylbenzoyl)-1-pipéridinyl]valéronitrile,
5-[4-(4-fluorobenzoyl)-1-piperidinyl]-2-dodécyl-2-(1-naphtyl)valéronitrile,

5-[4-(4-fluorobenzoyl)-1-pipéridinyl]-2-dodécyl-2-(2-naphtyl)valéronitrile,
2(3,4-diméthoxyphényl)-2-dodécyl-5-[4-(4-fluorophényl)méthylène-1-piperidinyl]valéronitrile,
5-[4-(4-fluorobenzoyl)-1-pipéridinyl]-2-dodécyl-2-(3-trifluorométhylphényl)valéronitrile,
5-[4-(4-fluorobenzoyl)-1-pipéridinyl]-2-dodécyl-2-(1-méthylpyrrol-2-yl)valéronitrile,
2-(3,4-dimethoxyphényl-5-[4-(α-hydroxy-4-fluorobenzyl)-1-pipéridinyl]-2-méthylvaléronitrile,
2-(3,4-diméthoxyphényl)-2-éthyl-5-[4-(α-hydoxy-4-fluorobenzyl)-1-pipéridinyl]valéronitrile,
2-(3,4-diméthoxyphenyl)-5-[4-(α-hydroxy-4-fluorobenzyl)-1-pipéridinyl]-2-propylvaléronitrile,
2-butyl-2-(3,4-diméthoxyphényl)-5-[4-(α-hydroxy-4-fluorobenzyl)-1-pipéridinyl]valéronitrile,
2-(3,4-diméthoxyphényl)-5-[4-(α-hydroxy-4-fluorobenzyl)-1-pipéridinyl]-2-pentylvaléronitrile,
2-(3,4-diméthoxyphényl)-2-hexyl-5-[4-(α-hydroxy-4-fluorobenzyl)-1-pipéridinyl]valéronitrile,
2-(3,4-diméthoxyphényl)-2-heptyl-5-[4-(α-hydroxy-4-fluorobenzyl)-1-pipéridinyl]-valéronitrile,
2-(3,4-diméthoxyphényl)-5-[4-(α-hydroxy-4-fluorobenzyl)-1-piperidinyl]-2-octylvaléronitrile,
2,-(3,4-diméthoxyphényl)-5-[4-(α-hydroxy-4-fluorobenzyl)-1-pipéridinyl]-2-nonylvaléronitrile,
2-décyl-2-(3,4-diméthoxyphényl)-5-[4-(α-hydroxy-4-fluorobenzyl)-1-pipéridinyl]valeronitrile,
2-(3,4-diméthoxyphenyl)-5-[4-(α-hydroxy-4-fluorobenzyl)-1-piperidinyl]-2-undécylvaléronitrile,
2-(3,4-diméthoxyphényl)-5-[4-(α-hydroxy-4-fluorobenzyl)-1-pipéridinyl]-undécylvaléronitrile,
2-(3,4-diméthoxyphenyl)-4-[4-(α-hydroxy-4-fluorobenzyl)-1-pipéridinyl]-butyronitrile,
2-(3,4-diméthoxyphényl)-6-[4-(α-hydroxy-4-fluorobenzyl)-1-pipéridinyl]-hexanenitrile,
2-(3,4-diméthoxyphényl)-7-[4-(α-hydroxy-4-fluorobenzyl)-1-pipéridinyl]-heptanenitrile,
2-(3,4-diméthoxyphényl)-8-[4-(α-hydroxy-4-fluorobenzyl)-1-pipéridinyl]-octanenitrile,
L-(+)-tartrate de 5-{3-[4-(4-fluorobenzoyl)-1-pipéridinyl]propyl}-2,3-dihydro-3-hydroxy-2-phényl-1,5-benzothiazepin-4(5H)-one,
L-(+)-tartrate d'acétate de 5-{3-[4-(4-fluorobenzoyl)-1-pipéridinyl]propyl}-2,3-dihydro-3-hydroxy-2-phényl-1,5-benzothiazépin-4(5H)-one,
4-[4-(4-fluorobenzoyl)-1-pipéridinyl]-(3',4'-diméthoxyphényl)butyrophénone,
4-[4-(5H-dibenzo[a,d]cycloheptén-5-ylidène)-1-pipéridinyl]-4'-fluorobutyrophénone,
2-(3,4-diméthoxyphényl)-2-isopropyl-5-(4-phényl-1-pipéridinyl)valéronitrile,
2,2-bis{3-[4-(4-fluorobenzoyl)-1-pipéridinyl]-1-propyl}-(3-méthoxyphényl)acétonitrile,
2-{3-[N-(3,4-diméthoxyphényl)-N-méthylamino]-1-propyl}-2-(3,4-diméthoxyphényl)-5-[4-(4-fluorobenzoyl)-1-pipéridinyl]valéronitrile,
chlorhydrate de 3-{2-[4-(4-fluorobenzoyl)-1-pipéridinyl]-1-éthyl]-5-(2-méthoxy-1-éthyl)-2,6-diméthyl-4-(3-nitrophényl)-1,4-dihydropyridine-3,5-dicarboxylate.

**5.** Utilisation selon l'une quelconque des revendications 1 à 4, dans laquelle le dérivé de l'éthylamine est utilisé en même temps qu'un excipient approprié.

**6.** Utilisation selon l'une quelconque des revendications 1 à 4, dans laquelle le dérivé de l'éthylamine est utilisé en même temps qu'au moins un agent parmi un diurétique, un antagoniste du calcium, un agent β-bloquant, un agent α-bloquant et un inhibiteur d'enzyme de conversion.